# EUROPEAN PATENT APPLICATION

(11) **EP 4 729 174 A2**
(43) Date of publication of application: **22.04.2026**
(21) Application number: 26150046.6
(22) Date of filing: 26.11.2021
(51) Int. Cl.: B01L 1/04

(54) **A DEVICE FOR PREPARING A DNA PRODUCT BY MEANS OF CAPILLARY POLYMERASE CHAIN REACTION**

(30) Priority: 27.11.2020 WO PCT/EP2020/083716
(62) Divisional of application: 21811399.1
(71) Applicant: CureVac Manufacturing GmbH, 72076 Tübingen (DE); Tesla Automation GmbH, 54595 Prüm (DE)
(72) Inventor: YAZDAN PANAH, Benyamin, 72076 Tübingen (DE); ROOS, Tilmann, 72076 Tübingen (DE); BUOB, Dominik, 72076 Tübingen (DE); GRACA, Telmo, 72076 Tübingen (DE); THOMMEN, Michael, 72076 Tübingen (DE); BERTSCH, Felix, 72076 Tübingen (DE); KOHLEN, Bernhard, 54636 Ingendorf (DE); RAUEN, Michael, 54595 Prüm (DE); SCHOLTES, Nico, 54347 Neumagen-Dhron (DE); HOFFMANN, Philipp, 54290 Trier (DE); WAGNER, Veronika, 72076 Tübingen (DE)
(74) Representative: Pinsent Masons LLP

(57) **Abstract**

The present invention relates to a device for preparing a DNA product by means of Capillary Polymerase Chain Reaction (PCR); a manufacturing device for a pharmaceutical product; a manufacturing module for a pharmaceutical product; a method for preparing a DNA product by means of Capillary Polymerase Chain Reaction; the use of the device for preparing a DNA product by means of Polymerase Chain Reaction; and the use of the device for a production of a pharmaceutical product.

The device for preparing a DNA product by means of Capillary Polymerase Chain Reaction comprises a tube for guiding a PCR liquid, a first compartment, and at least second compartment. The tube is wound at least from the first compartment to the second compartment and from the second compartment to the first compartment, wherein the tube wound from compartment to compartment forms a helical stack of tube windings. The first compartment and the second compartment each comprises means to adjust the temperature in the compartment to prepare the DNA product based on the PCR liquid, wherein the first compartment is configured to provide a temperature for denaturation and the second compartment is configured to provide a temperature for annealing and/or elongation.

## Description

### TECHNICAL FIELD

The present invention relates to a device for preparing a DNA product by means of Capillary Polymerase Chain Reaction; a manufacturing device for a pharmaceutical product; a manufacturing module for a pharmaceutical product; a method for preparing a DNA product by means of Capillary Polymerase Chain Reaction; the use of the device for preparing a DNA product by means of Polymerase Chain Reaction; and the use of the device for a production of a pharmaceutical product.

### BACKGROUND OF THE INVENTION

Active pharmaceutical ingredients (APIs) in the form of biomolecules are used today largely in therapies of a variety of diseases. Such biomolecules are e.g. therapeutic antibodies, peptides and nucleic acids. Therapeutic nucleic acids including DNA molecules and RNA molecules.

RNA represents an emerging class of drugs. RNA-based therapeutics include mRNA molecules encoding antigens for use as vaccines. For the manufacturing of RNA, a DNA template is typically used in an RNA in vitro transcription reaction. Such a DNA template may be obtained by polymerase chain reaction (PCR). Accordingly, the production of DNA in the meaning of amplification of DNA by PCR is a critical step for the manufacturing of RNA as API.

Moreover, DNA as such may serve as API, for example DNA vaccines against Coronavirus are currently under clinical development. For the manufacturing of DNA in large amounts, i.e. for the amplification of DNA, a polymerase chain reaction (PCR) may be used.

Currently established manufacturing processes for APIs in the form of nucleic acids approved by regulatory authorities typically implement many separate manufacturing steps, all of which must comply with GMP-standards. Such steps are typically carried out in GMP-compliant rooms that are present in production plants and thus not portable. Furthermore, the rooms are typically set up for the production of a specific biomolecule and are thus not flexible but need to be rebuilt to a rather large extent if a different nucleic acids needs to be produced. In summary, the manufacturing of nucleic acids at API-grade requires a large degree of manual handling in a GMP-regulated, rather inflexible and fixed laboratory, executed by well-trained technical staff. In consequence, current established manufacturing processes are time consuming, cost intensive, and require a lot of laboratory space and laboratory equipment.

### SUMMARY OF THE INVENTION

As outlined above, there is the problem associated with common manufacturing processes for nucleic acids, in particular DNA and RNA, as APIs or intermediates during the production process of APIs that the processes are rather inflexible, not portable and typically require a large degree of manual handling of well-trained technical staff. Thus, there is a need for providing an improved device for preparing/amplifying in particular DNA and a corresponding manufacturing device comprising such a device, which is more flexible. The manufacturing device should in particular provide the benefits to be operated under GMP-compliant conditions, to be portable and/or to be automated. Such a manufacturing device would reduce the time for manufacturing the API (e.g. DNA) or an intermediate (e.g. DNA that may serve as template for RNA production, wherein the RNA is the API) tremendously which is particularly important in the context of an outbreak of a virus (e.g. pandemic outbreak or local outbreak). Such a portable API / intermediate manufacturing device (comprising the device for preparing/amplifying in particular DNA, which may be referred to as "PCT reactor") could be easily shipped, installed and operated in pandemic hotspot regions around the globe and would therefore allow for a rapid production of a vaccine in said region. Therefore, it is of paramount importance that the manufacturing device of the invention is configured to produce nucleic acids APIs / intermediates under GMP-compliant conditions, and that a corresponding "PCR reactor" is configured to be operable in such a manufacturing device.

The above problem is solved by the subject-matter of the independent claims, wherein further embodiments thereof are provided in the dependent claims.

In **a first aspect,** the present invention is directed to a device for preparing a DNA product by means of Capillary Polymerase Chain Reaction (PCR). The "preparing a DNA product by means of Capillary PCR" may alternatively be referred to as "amplifying DNA by Capillary PCR" or "amplifying DNA by PCR". The device comprises a tube for guiding a PCR liquid, a first compartment, and at least a second compartment. The tube is wound at least from the first compartment to the second compartment and from the second compartment (back) to the first compartment, wherein the tube wound from compartment to compartment forms a helical stack of tube windings. Typically, each tube winding represents one PCR cycle. The first compartment and the second compartment each comprises means to adjust the temperature in the compartment to prepare the DNA product based on the PCR liquid, wherein the first compartment is configured to provide a temperature for denaturation and the second compartment is configured to provide a temperature for annealing and/or elongation.

In preferred embodiments, the device for preparing a DNA product by means of Capillary Polymerase Chain Reaction (PCR) is not a microfluidics device or dimensioned to operate as a microfluidics device. In preferred embodiments, the device for preparing a DNA product by means of Capillary Polymerase Chain Reaction (PCR) is not dimensioned or configured to operate as an analytical PCR device. In preferred embodiments, the device for preparing a DNA product by means of Capillary Polymerase Chain Reaction (PCR) is dimensioned or configured to operate as an device for the preparative production of DNA (e.g. for the production of more than 1 mg DNA product, optionally even for the production of DNA in g-amounts, e.g. the production of 5 g DNA). If run in a continuous mode, the output is essentially unlimited such that e.g. a very large amount of DNA in g-amounts can be provided, in particular DNA serving as template DNA in a subsequent RNA in vitro transcription reaction when the overall aim is to produce RNA.

In an embodiment, the device is not constructed such that a recirculation of the PCR liquid inside the device and/or tube comprised in the device is possible but rather, as noted above, the tube is wound in a helical stack of tube windings, wherein the tube is at no position reconnected to a previous section of the tube (accordingly, the PCR liquid enters the tube at the first end of the tube, stays inside the tube during all PCR cycles and leaves the tube at the second end with DNA product having been formed). In an embodiment, the device does not comprise a closed loop (which may e.g. be formed by the tube or by reaction tanks connected via the tube) for the PCR liquid. In an embodiment, the device does not comprise a closed loop for the PCR liquid connected to a valve, wherein the valve would allow the flow of the PCR liquid and the DNA product, respectively, into a separate tube exiting the device.

The DNA product can be DNA that corresponds to the API (e.g. a DNA vaccine) or a DNA template (and thus an intermediate for RNA as the API) for enzymatic RNA in vitro transcription. RNA in vitro transcription relates to a process wherein RNA is synthesized in a cell-free system. The DNA template is a DNA molecule comprising a nucleic acid sequence encoding the RNA sequence to be transcribed in vitro. The DNA template is used as a template for RNA in vitro transcription in order to produce RNA encoded by the template DNA. The DNA template can be transcribed into an mRNA representing the API, which can be formulated into a lipid nanoparticle resulting in encapsulated mRNA, which may further be formulated into a final drug product. Accordingly, the DNA product may be the active pharmaceutical ingredient (as in a DNA vaccine), or the DNA product may be a DNA template that can be transcribed into an mRNA as the active pharmaceutical ingredient (such that the DNA is not the API but an intermediate). A DNA product in the context of the invention is DNA obtained by PCR, i.e. amplified DNA.

Polymerase Chain Reaction (PCR) is one of the fastest ways to prepare a DNA product. Typically, PCR can be performed in PCR devices for laboratory use and scale. It is a technology in molecular biology to synthetically amplify DNA across several orders of magnitude, generating thousands to millions of copies of a particular DNA sequence. PCR relies on thermal cycling comprising cycles of repeated heating and cooling for DNA melting and enzymatic replication of the DNA using thermostable DNA polymerases. The cycles comprise a denaturation temperature, an annealing temperature, and an elongation temperature. The separate temperatures in the first compartment and the second compartment may be one of the denaturation temperature, the annealing temperature and/or the elongation temperature. Specific PCR techniques comprise for example RT-PCR, Hot-Start PCR, Long-Range PCR, RT-qPCR, etc.

Capillary Polymerase Chain Reaction can be understood as PCR done in a tube, a pipe, or a capillary. The tube or capillary can be very thin or fine. The tube or capillary may have a very small diameter, which is in a range of about 0.5 to about 50 mm, preferably about 0.5 mm to about 1.5 mm, more preferably about 0.75 to about 1.25 mm. The diameter of the capillary or tube is suitably configured to allow a fast and efficient temperature change of the PCR liquid in the tube, when the PCR is ongoing, i.e. when the PCR liquid is flowing / progressing through the tube during the PCR. As described herein, the tube is passing through different compartments that are configured to provide different temperatures such that the tube and thereby the PCR liquid inside the tube is exposed to these different temperatures. The tube or capillary and in particular its diameter (or outer dimension) may be configured to allow a capillary action or capillary effect with the PCR liquid. Capillary action or capillary effect is the ability of a liquid to flow in narrow spaces without the assistance of, or even in opposition to, external forces like gravity. The capillary action or capillary effect can be seen in the drawing up of liquids e.g. in a thin tube, in porous materials such as paper, in some non-porous materials such as sand, etc. It occurs because of intermolecular forces between the liquid and surrounding solid surfaces. If the diameter of the tube or capillary is sufficiently small, then a combination of surface tension which is caused by cohesion within the liquid) and adhesive forces between the liquid and a tube or capillary wall act to propel the liquid.

The tube is wound at least from the first compartment to the second compartment and from the second compartment (back) to the first compartment. "Wound" can be understood as not forming a line (segment) being the shortest distance between two points. Instead, the wound tube can be longer than the shortest distance between two points. It may extend in a round, curved or wavy shape. The reason for the tube being wound is that the length of the tube in a specific compartment together with a pre-defined flowrate determine the exposure time of the PCR liquid to the specific temperature inside a compartment. This in turn defines how long the PCR liquid inside the tube is exposed to specific temperatures required for a PCR, e.g. a denaturation temperature, which e.g. may be present in the first compartment. If the tube passes the first compartment in the shortest way to the second compartment, the tube length exposed to the denaturation temperature provided in the first compartment may be too short, ultimately resulting in the DNA not sufficiently denatured. If, however, the tube passes the first compartment in a wound way, the length of the tube exposed to the denaturing temperature provided in the first compartment is sufficiently long, such that PCR liquid passing through this tube section is exposed to this temperature for a sufficient period, ultimately resulting in the DNA being sufficiently denatured.

It is important to understand that the tube according to the present application does not connect one (or more) reaction-mixture tank(s), in particular not one reaction-mixture tank. In other words, the tube according to the present application does not allow for a recirculation path, where the tube starts from a reaction-mixture tank, passes through different tanks (of different temperatures) and then comes back to the very same reaction-mixture tank in order to provide for a recirculation. Furthermore, it is important to understand that the tube is not connected to a valve, which would enable a recirculation path.

The PCR liquid can be understood as a reaction solution comprising a DNA template for a PCR amplification (wherein the DNA template is initially present in a small amount; it can be preferred that this initial DNA template for a PCR amplification is de-novo-synthesized DNA), one or more DNA primers, dNTPs, a DNA polymerase (e.g. a proofreading DNA polymerase), and a suitable PCR buffer. The PCR liquid is filled or pumped into the tube at one end (wherein the tube comprises overall two ends: an "inlet end" for the starting PCR liquid and an "outlet end" for the resulting amplified DNA product) and guided through the compartments by the tube wound in and through these compartments. The PCR liquid is separated by the tube walls from the area inside the compartments and thus e.g. from thermal medium (e.g. a liquid or a gas) or a thermal solid to be filled in the compartments such that the PCR liquid at no point has any direct contact with the compartments and the space inside the compartments, respectively. The tube walls allow for a temperature exchange between the PCR liquid and the compartments (and thus the temperatures inside the compartments), e.g. the thermal medium or the thermal solid inside the compartments, which are thus used to heat or cool the PCR liquid by the means to adjust the temperature of the tube, e.g. by the thermal medium or a thermal solid. While the PCR liquid is guided through the compartments, the template for PCR amplification is amplified and a DNA product is generated. This DNA product is then exiting the tube at the second end of the tube, the "outlet end". Accordingly, the DNA product does not need to be taken from a reaction-mixture tank or any corresponding entity in recirculation system.

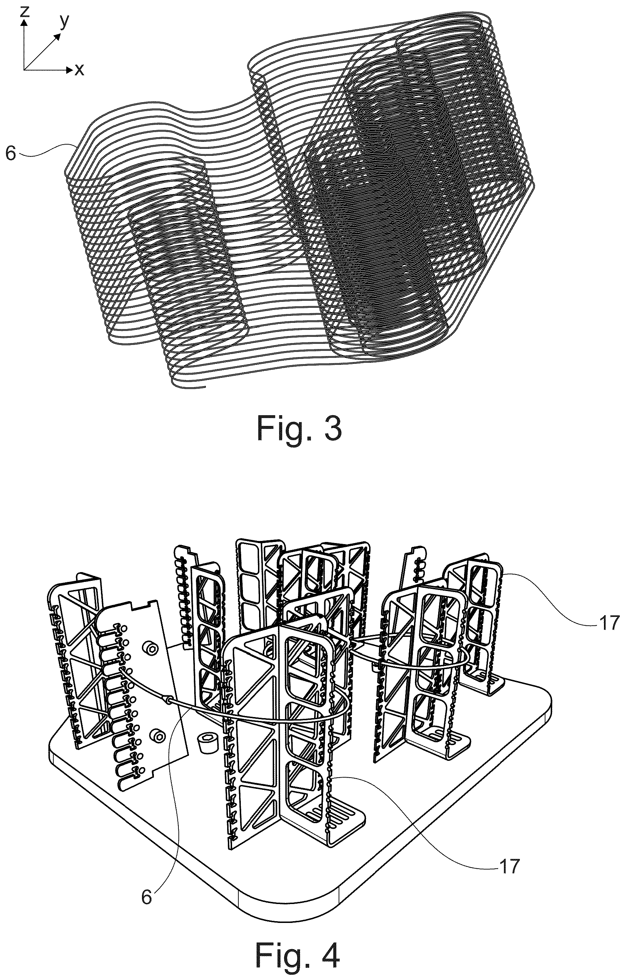

The tube forms a helical stack of tube windings (essentially as exemplarily shown in Figure 3). A single "tube winding" means "one circle" of the tube back to the starting first compartment, namely that the tube is wound from the first compartment to the second compartment and from the second compartment (back) to the first compartment or, preferably, through the first compartment and from the first compartment to and through the second compartment, from the second department to and through the third compartment, and from the third compartment (back) to the first compartment. It is important to understand that the tube goes back to the first compartment without, however, connecting back to the initial section of the tube in the first department. Instead, the tube, after having made a tube winding from the first compartment through the compartments and back to the first compartment, continues by now being wound in a helical form ultimately forming a helical stack. In other words, the tube does not form a "two-dimensional" circulation from the first compartment through the different compartments back to the first compartment, where it is connected to the initial tube again thus forming a circulation, but the tube forms a "three-dimensional" helix from the first compartment through the different compartments back to the first compartment without connecting to the initial tube. The helix in the "third dimension" can in particular be essentially perpendicular to each winding in the "second dimension". This is what is meant by the "stacking direction of the helical stack of tube windings being different and preferably essentially perpendicular to a corrugation direction of the corrugated tube". The helical stack of the tube windings allows for multiple PCR-cycles, wherein each winding corresponds to a single PCR cycle.

The tube comprises two distinct ends, a "PCR liquid inlet" and a "DNA product outlet", wherein the first end is typically located at the tube before the tube enters the first compartment (this is where the PCR liquid enters the device) as the denaturation step is the first step of the PCR reaction and the second end is typically located at the tube after the tube leaves the second/third compartment (this is where the DNA product leaves the device) as the elongation step is typically the last step of the PCR reaction.

The device for preparing a DNA product comprises at least two (tempering) compartments, a first compartment and a second compartment. There can also be a third compartment, which is typically preferred. Even more compartments are possible. The compartments can be understood as at least partially and preferably fully closed space, room or container. The compartments may have a volume of at least 10 mL, preferably at least 100 mL, more preferably at least 1 L, for example about 1 L to about 10 L. They can be sealed and leak-tight. The at least two compartments may form at least two independent or different basins to be filled e.g. with thermal medium or a thermal solid. The at least two compartments may be separated from each other and preferably thermally insulated from each other to allow separate, different or independent temperatures zones in each of the at least two compartments. These temperatures zones correspond to the temperature profiles of the PCR. It is particularly important for the efficiency of a PCR to have fast "ramp rates" which indicates the change in temperature over time. In the context of the present invention, the change in temperature over time is determined by the flow rate of the PCR liquid and the time required for heat exchange in the compartments.

It is an option to adjust the temperature in each compartment by thermal medium. The thermal medium may be a liquid or a gas. If so, the thermal medium may enter in an embodiment each of at least two compartments by a respective thermal medium inlet and may exit each of the at least two compartments by a respective thermal medium outlet. Accordingly, in this embodiment, each compartment comprises a thermal medium inlet and a thermal medium outlet. There can thus be a flow of thermal medium through the respective basin in the at least two compartments, which are filled at least partially by the thermal medium. The thermal medium can be understood as a liquid coolant and heat carrier, e.g. on basis of highly heat-conducting media like e.g. propylene glycol, silicone-based fluids or other synthetic thermal media, as well as water or any mixture of the preceding liquids. The thermal medium can also be understood as a gaseous coolant or gaseous heat carrier. The thermal medium can be e.g. Pekasol^{®}. The thermal medium may be used to provide, control and/or adjust separate temperatures in the at least two compartments to prepare the DNA product by means of the thermal cycling being part of the PCR. The thermal medium in the different compartments is configured to allow a fast temperature change of the tube and thus of the PCR liquid inside the tube during the PCR. In an embodiment, a compartment does not comprise stirring means to mix the thermal medium in the compartment. In another embodiment, the compartment comprises stirring means to mix the thermal medium in the compartment.

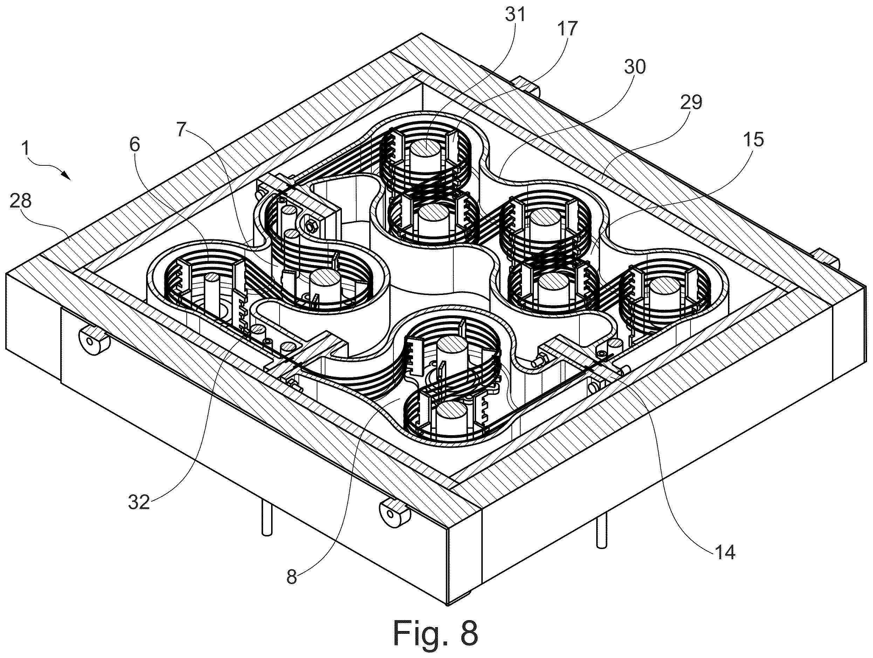

It is another option to adjust the temperature in each compartment by a thermal solid. If so, the temperature of the thermal solid is provided and adjusted in this embodiment by a heating unit and optionally a cooling unit. In this embodiment, liquid thermal medium is not used and, accordingly, a thermal medium inlet and thermal medium outlet are not present in the compartment. The thermal solid is a solid that is thermally conductive and thus provides a specific temperature to the compartment and tube, respectively, that is enclosed by the thermal solid (the thermal solid is configured to allow a fast temperature change of the tube and thus of the PCR liquid inside the tube during the PCR). The thermal solid can be selected from the group consisting of aluminum, an aluminum alloy, or other alloys with high thermal conductivity, copper, bronze and brass, wherein aluminum or an aluminum alloy is preferred. The thermal solid may have the form of pellets, balls and/or a coarse powder, preferably with a diameter of about 1 to about 3 mm for the pellets and balls. It is particularly preferred to use aluminum pellets. The thermal solid may be comprised in a liquid or a gel in order to remove the residual air in the compartment. Accordingly, in this setup, the compartment (or the part of the compartment that is formed by the inner-compartment wall (30), see Figure 8) enclosing the tube is filled with a thermal solid as outlined above and a liquid or a gel, and is devoid of air. The heating unit is preferably an electric heating unit. Suitably, the heating unit is integrated in the stand (or tube holding means) to allow an optimal heating. The electric heating unit inside the compartment may e.g. be a heating cartridge or a heating wire or a heating conductor. By using in addition a cooling unit, in particular a thermoelectric cooler (i.e. a Peltier element) with a heat pipe connected thereto, wherein the heat pipe is extending into the compartment and thus also present inside the compartment, it is furthermore possible to cool the compartment (or the part of the compartment that is formed by the inner-compartment wall (30, see Figure 8) in order to provide and/or adjust the required temperature. The thermoelectric cooler may also be used to provide additional heat to the compartment, if it is run in the heating mode that is the opposing mode to the cooling. If run in a cooling mode, a ventilator may also be present to remove the heat of the thermoelectric cooler. However, the presence of a ventilator is not mandatory as the heat derived from the cooling may also be used as additional heating in (another) compartment of the device. Typically, several independent electronic heating systems are present in each compartment, wherein each of these systems is connected to its own temperature sensor to achieve several independent control circuits.

The device according to the present invention for preparing a DNA product allows for a flexible adaption to different conditions, requirements and e.g. quantities of DNA. The present device may allow a preparation of large quantities of DNA (e.g. more than 2 mg per reaction). Further, the preparation of a DNA product may be scalable. In particular, the preparation of a DNA product may be continuous, where g-amounts and essentially unlimited amounts may be produced as new PCR liquid is continuously added in the "inlet" while resulting DNA leaves the device through the "outlet" of the tube and device, respectively. The present device for preparing a DNA product allows for a very fast preparation of a DNA product and thereby also a fast manufacturing of a pharmaceutical product or an intermediate. Also a change and adaption of the device for preparing a DNA product to a specific DNA product may be very fast. There may be no need for adjustment or calibration steps. There may be no or fewer need for cleaning steps. The turnover time for e.g. a vaccine manufacturing may be less than about a week.

The device according to the present invention for preparing a DNA product is constructed in a very elegant manner. It can be dimensioned to be very small. The present device for preparing a DNA product by means of Capillary PCR is portable to allow transportation to e.g. regions of an outbreak of a pandemic. It may thereby allow a local, decentral production of a pharmaceutical product (e.g. a DNA vaccine) or of an intermediate (e.g. DNA template) for a pharmaceutical product (e.g. an RNA vaccine).

The device according to the present invention for preparing a DNA product may be used for a flexible and fast DNA vaccine production or a DNA template production for an RNA vaccine, particularly a flexible and fast RNA vaccine production. The present device may be used in particular for the production of DNA templates for mRNA-based vaccines e.g. during infectious disease epidemics and pandemics as well as for diverse cancer diseases including personalized therapies.

In an embodiment, septum walls are arranged between the compartments to insulate the compartments from each other. This can be understood in that a septum wall is arranged between the first compartment and the second compartment. In case there are more, e.g. three compartments, a septum wall may be arranged between the first compartment and the second compartment and another septum wall may be arranged between the second compartment and the third compartment and/or a further septum wall may be arranged between the third compartment and the first compartment. As a consequence, the (adjacent) compartments may be thermally insulated from each other and therefore different temperatures can be established and maintained in the (adjacent) compartments. The septum walls may be implemented by only one wall component to insulate all compartments by the same wall component or by more than one wall component. A septum wall may be of a material with low thermal conductivity and with good mechanical and temperature resistance. Additionally or in part as an alternative, an inner-compartment wall may be present that more closely follows the corrugated tube and thus creates a narrower space surrounding the tube in each compartment. This inner-compartment wall also results in an insulation of the respective compartment, while creating a rather narrow space around the tube to be filled in particular with a thermal solid. If an inner-compartment wall is present, the septum walls are not necessarily fully present between the compartments but may only be present partially, in particular at the area, where the tube passes from one compartment to the next compartment throught a septum wall as described next.

Thus, in a most preferred embodiment, the septum walls comprise through holes for the tube to extend from one compartment to the next compartment such that the tube is able to pass through the different compartments. The through holes or channels enable the tube to enter and/or exit a compartment and its temperature zone. The through holes may be sealed to improve a leakage tightness. The through holes may also be thermally insulated to improve temperature stability in (adjacent) compartments.

In an embodiment and if present, the thermal medium inlet and the thermal medium outlet are arranged within at least one and preferably within each compartment to provide the thermal medium flow through the compartment essentially in a counter direction to a guiding direction of the PCR liquid in the tube. This can be understood that in case of a clockwise guiding direction of the PCR liquid, the thermal medium inlet and the thermal medium outlet are arranged to provide the thermal medium flow in a counter clockwise direction or vice versa. Or, in case of a guiding direction of the PCR liquid from a left side to a right side, the thermal medium inlet and the thermal medium outlet are arranged to provide the thermal medium flow in a direction from a right side to a left side or vice versa. The counter flow of thermal medium and PCR liquid improves a temperature exchange between the thermal medium and the PCR liquid and thereby a temperature control of the PCR liquid. It is particularly important for an efficient PCR reaction to have constant temperatures in each of the compartments of the device, and to facilitate a fast temperature exchange when the tube comprising the PCR liquid is guided into the next compartment (that typically has a different temperature).

In an embodiment, the thermal medium inlet, if present, is arranged at a lower position in the respective compartment and the thermal medium outlet, if present, is arranged at an upper position in the respective compartment. Such a configuration has the advantage that gas bubbles may be removed more easily.

Alternatively, the thermal medium inlet, if present, may be arranged at an upper position in the respective compartment and the thermal medium outlet, if present, may be arranged at a lower position in the respective compartment.

In an especially preferred embodiment, the device for preparing a DNA product by means of Capillary Polymerase Chain Reaction further comprises a third compartment. The tube may be wound at least (through and) from the first compartment to (and through) the second compartment and from the second compartment to (and through) the third compartment. The tube may be further wound from the third compartment to the first compartment, again (through and) from the first compartment to (and through) the second compartment, and from the second compartment to (and through) the third compartment. Also the third compartment comprises means to adjust the temperature in the compartment. In this respect, it may comprise a thermal medium inlet and a thermal medium outlet to provide a thermal medium flow through the third compartment which is separate to the thermal medium flows through the first compartment and the second compartment. The separate thermal medium flow may be used to adjust a separate temperature in the third compartment, which is separate to the temperatures in the first compartment and the second compartment. Alternative to the thermal medium inlet and outlet, the embodiment with the heating unit as described above may be used as means to adjust the temperature in the compartment. Also the separate temperature in the third compartment is used to prepare the DNA product based on the PCR liquid. The three temperatures zones typically correspond to the temperature profiles of the PCR for denaturation, annealing, and elongation.

The tube wound from compartment to compartment forms a helical stack of tube windings. As defined above, a tube winding is a section of the tube that starts from the first compartments and comes back to the first compartment, wherein a tube winding represents one PCR cycle. Such tube windings then form a helix, ultimately resulting in a helical stack of tube windings, wherein each tube winding represents one PCR cycle. In other words, this can be understood in that the tube has the shape of a helix, spiral or coil (when seen in a side view). In still other words, the tube may run in circles (which are, however, not connected to each other), which makes the device for preparing a DNA product by means of Capillary Polymerase Chain Reaction very elegant and space efficient. A circle may run from the first compartment to the second compartment and from the second compartment to the (optional) third compartment. Every circle corresponds to a PCR cycle. The helical extension or stacking direction of the tube may extend in a so-called z-direction away from a so-called x, y plane formed by a bottom of the device for preparing a DNA product or a plane formed by the floor or ground. The helical extension of the tube may extend essentially perpendicular, but also angular to the bottom of the device, the floor or ground.

In an embodiment, the tube extends within at least one and preferably in each of the compartments in a corrugated or meandering manner resulting in a specific length of the tube in each compartment, reflecting the incubation time of the PCR liquid inside the tube when passing through the tube inside this compartment preferably with a pre-defined and controlled flow rate. As this incubation time is typically longer than the time achieved if the tube would be linear inside a compartment, it is preferred that the tube is present in each compartment in a corrugated or meandering manner. This can be understood in that the tube has the shape of a wave or a snake within one or all of the compartments (when seen in a top view). The wave or snake shape or corrugation of the tube may extend in x- and y-directions of the plane formed by the bottom of the device for preparing a DNA product or the plane formed by the floor or ground, i.e. it is to be understood as relevant for the "two-dimensional" form and does not include the helical, i.e. the "three-dimensional" form. The corrugated form of the tube defines a dwell time of the PCR liquid in the respective compartment and thereby defines a dwell time of the PCR liquid in the temperature zone provided by the respective compartment. Accordingly, the corrugated form of the tube defines the length of the tube in the respective compartment having a certain temperature and thereby defines a dwell time of the PCR liquid in the temperature zone provided by the respective compartment.

In an embodiment, the stacking direction of the helical stack of tube windings is different and preferably essentially perpendicular to the corrugation direction of the corrugated tube. The angle between the stacking direction of the helical stack of tube windings and the corrugation direction of the corrugated tube may also be different to about 90°, e.g. below about 90°, in a range of 45° to 89°. The stacking direction of the helical stack of tube windings and the corrugation direction of the corrugated tube may then be inclined or oblique to each other. The stacking direction of the helical stack of tube windings may be inclined or oblique relative to the bottom of the device for preparing a DNA product or the plane formed by the floor or ground. Additionally or alternatively, the corrugation direction of the corrugated tube may be inclined relative to the bottom of the device for preparing a DNA product or the plane formed by the floor or ground. The tube may then have a pitch relative to the bottom of the device for preparing a DNA product or the plane formed by the floor or ground. The pitch may be the same throughout the device for preparing a DNA product or throughout one of the compartments, but the pitch may also vary throughout the device for preparing a DNA product or throughout one of the compartments.

In an embodiment, the device for preparing a DNA product by means of Capillary Polymerase Chain Reaction further comprises at least a stand or tube holding means arranged in one of the compartments to provide a fixation point for the tube and/or a deviation point for a corrugation of the tube. The stand may have a cylindrical shape. It may also have a T-shape or an L-shape, when seen in a top view. Also other shapes are possible. The stand may comprise at least one mounting structure configured to receive and fix the tube (and thus the tube windings and helical stack of tube windings, respectively). Preferably, the stand comprises a plurality of mounting structures to fixedly hold the tube (and thus the tube windings and helical stack of tube windings, respectively) at the stand. The mounting structure may be formed as a recess, as a through-hole, as a hook, as a slit or the like or other means to mount the tube (and thus the tube windings and helical stack of tube windings, respectively) to the stand. The tube may be fixed at the mounting structures in a force-fitting and/or a form fitting manner. Additionally or alternatively, the stand may further comprise at least one clamping means to fixedly hold the tube. Preferably, the mounting structure is coated with a soft material comprising e.g. a rubber or silicone to prevent the tube from damages such as scratch, cut, etc. Suitably, the stand may be of a material that has a chemical resistance to the used thermal medium.

In an embodiment, the tube extends partially around an outer dimension of the stand. This can be understood in that, in a top view, the tube extends not fully, but less than about 360° around the outer dimension or circumference of the stand. It may extend in a range of about 45° to about 270° around the outer dimension of the stand. In another embodiment, the tube extends fully around an outer dimension of the stand. This can be understood in that, in a top view, the tube extends about 360° around the outer dimension or circumference of the stand. In another embodiment, the tube extends more than once around an outer dimension of the stand. This can be understood in that, in a top view, the tube extends more than about 360° around the outer dimension or circumference of the stand. It may extend in a range of about 450° to about 540° around the outer dimension of the stand, this means, there might be an overlap of tube around the stand.

In an embodiment, the stand is exchangeable in the compartment. This can be understood in that the stand is realisably fixed to the compartment and particular to a bottom plate of the compartment. As a consequence, the stand can be exchanged by another stand with e.g. different outer dimensions (radius and/or height) to adapt the device for preparing a DNA product to different tubes, different purposes, different PCR durations (e.g. to reflect a different PCR protocol), a different winding radius, different winding patterns and/or the like.

In an embodiment, the stand is repositionable in the compartment. This can be understood in that the stand can be positioned at different positions or locations within the compartment and particular at different positions relative to the bottom plate of the compartment. Therefore, the bottom plate maybe formed as a perforated plate with (blind) holes, in which the stand can be flexibly inserted. As a consequence, the stand can be repositioned to adapt the device for preparing a DNA product to different tubes, different purposes, different PCR durations (e.g. to reflect a different PCR protocol), a different winding radius, different winding patterns and/or the like.

The stand and in particular the exchangeable and/or repositionable stand can be used to define a dwell time of the PCR liquid in the compartments and thereby a dwell time of the PCR liquid in the temperature zones provided by the compartments. Hence, the dimensions and positions of the stands may define a temperature profile of the PCR and can be flexibly adjusted to match desired PCR profiles (e.g. for producing different DNA products e.g. DNA products of different length).

The tube may be continuous throughout the device for preparing a DNA product, which can be understood as being the same throughout the device for preparing a DNA product. The tube may also be discontinuous throughout the device for preparing a DNA product, which can be understood as e.g. comprising different portions with e.g. different sizes or diameters in a cross section of the tube. The tube may be exchangeable to adapt the device for preparing a DNA product to different PCRs or different other purposes. For cleanability reasons, the tube is preferably continuous. Further, the tube is configured to allow corrugation of the tube e.g. via the stand, without the danger of breaking or bending down which could impede the efficiency of the PCR. However, in all instances, the tube is not a recirculation or closed tube system with an optional outlet but comprises a defined "inlet" end and a defined "outlet" end.

In an embodiment, the tube comprises a PCR liquid inlet, which is connectable to a pump unit for pumping the PCR liquid through the tube. The pumping unit may be a pump. The pumping unit may be an external pumping unit. In an embodiment, the pump unit is arranged outside the compartments and preferably outside a shell surrounding the compartments. The shell may be made of a plastic material, as e.g. of POM, PEEK, PTFE, but also out of metal, an alloy, a compound or the like and being resistant and compatible to heat, and thermal media applied. The external arrangement may save space within device for preparing a DNA product and/or may allow a use of a powerful pumping unit. Preferably, the pumping unit comprises a micro gear pump configured to generate a constant flow rate.

In an embodiment, the pump unit is configured to provide a flow rate of the PCR liquid in a range of about 0.05 mL/min to about 50 mL/min, preferably about 0.1 mL/min to about 10 mL/min, more preferably about 0.2 mL/min to about 3 mL/min. Such flow rates may define a dwell time of the PCR liquid in the compartments and thereby a dwell time of the PCR liquid in in the temperature zones provided by the compartments. Hence, these flow rates may define a temperature profile of the PCR. Preferably, the flow rate is constant, e.g. the flow rate fluctuates less than +/- 10%, preferably less than +/- 5%.

In an embodiment, the tube comprises a DNA product outlet for a continuous preparation of the DNA product. This can be understood in that there is a continuous, non-stop preparation of the DNA product. The DNA product outlet can therefore be "always open during production". Such continuous preparation of the DNA product improves a continuous and scalable production of large quantities of DNA product. The DNA product outlet may comprise a means of a closing mechanism as e.g. a shutter that can be closed when the continuous preparation of the DNA product is finalized.

In an embodiment, the tube comprises a closable DNA product outlet for a discontinuous preparation of the DNA product. This can be understood as a discontinuous, batch-per-batch preparation of DNA product. The DNA product outlet is therefore closable by means of a closing mechanism as e.g. a shutter.

In an embodiment, the device for preparing a DNA product by means of Capillary Polymerase Chain Reaction further comprises at least a sensor arranged in at least one of the compartments. The sensor may be a temperature sensor, a flow rate sensor, a leakage sensor and/or the like, which is/are respectively configured to detect a temperature, a flow rate, a leakage and/or the like of the compartment. The flow sensor may form a closed loop control with the pumping unit to monitor and control the flow rate outside the device for preparing a DNA product or PCR reactor. In at least one and preferably in each compartment, at least two temperature sensors may be provided, e.g. one near a fluid inlet, one near a fluid outlet for building a redundancy. Two leakage sensors may be used, one inside at least one compartment and preferably in each compartment, and one inside a surrounding unit/frame. Further sensors may be arranged outside the compartments, e.g. in the pump unit, or in the PCR liquid inlet, or in the DNA product outlet.

The device may also comprise the first part of a proximity sensor, e.g. a magnetic sensor, which finds its counterpart or second part of the proximity sensor outside the device according to the first aspect. In view of this, this second part of the proximity sensor may be located in particular in the manufacturing device according to the second aspect. Once the device has been installed properly in the manufacturing device, the two parts of the proximity sensor are connecting and preferably signaling that the installing was successful and/or preferably only then the device can successfully be started and operated. Accordingly, the proximity sensor comprising the two parts may also be active during operation and result in an alarm and/or stop of the operation once the two parts of the proximity sensor lost their connection.

In an embodiment, the tube has a diameter (which may correspond roughly to the outer dimension) in a range of about 0.5 mm to about 50 mm, about 0.5 mm to about 40 mm, preferably about 0.5 mm to about 20 mm, preferably about 0.5 mm to about 4 mm, more preferably about 0.5 mm to about 1.5 mm, even more preferably about 0.75 mm to about 1.25 mm. These dimensions may allow fast and efficient temperature exchange ("ramping") between the thermal medium and the PCR liquid.

The tube may be made of a plastic material, as e.g. PEEK, ETFE or PTFE but also out of metal, an alloy, a compound or the like. Suitably, the tube may be made of a material that is bio- and process-compatible (e.g. no leakage of compounds into the DNA product, no oxidation/degradation of the material, low association of the PCR compounds to surfaces to minimize risk of deprivation). A preferred material may be PEEK.

In an embodiment, the tube has a length between the tube inlet and the tube outlet in a range of about 5 m to about 300 m, preferably about 10 m to about 200 m, more preferably about 25 m to about 50 m. It can e.g. have a length of about 100 m (e.g. about 97 m). Sections of the tube are comprised in the different compartments, and these dimensions may define a dwell time of the PCR liquid in the compartments and thereby a dwell time of the PCR liquid in the temperature zones provided by the compartments. Hence, these tube length sections may define a temperature profile of the PCR.

In an embodiment, the tube is dimensioned to contain PCR liquid in a range of about 10 mL to about 1L, preferably about 20 mL to about 250 mL, more preferably about 30 mL to about 150 mL. It may e.g. be dimensioned to contain PCR liquid in the range of about 80 mL.

In an embodiment, the helical stack of tube windings comprises about 10 to about 50 windings or circles or layers, preferably about 15 to about 40, more preferably about 20 to about 30. The number of windings corresponds to the number of cycles of the PCR.

Merely as an example, the tube may have a total length of about 15 m, comprising about 25 helical stacks (or cycles) of tube windings. In that specific embodiment, in one helical stacks (or cycle) the tube may be guided through a first compartment (100cm) followed by a second compartment (100cm) followed by a third compartment (400cm).

In an embodiment, the device is configured to prepare about 1 mg and more of the DNA product per reaction, such as e.g. up to 5 g, preferably in a range of about 1 mg to about 30 mg, more preferably in a range of about 15 mg to about 25 mg.

In an embodiment, the device is configured to prepare in a range of about 2 µg/min to 2000 µg/min DNA product, preferably about 5 µg/min to 1500 µg/min DNA product, more preferably about 10 µg/min to 1000 µg/min.

In an embodiment, the device is configured to prepare the DNA product in a range of about 25 µg to about 200 µg per mL PCR liquid, preferably about 50 µg to about 150 µg per mL PCR liquid, more preferably about 75 µg to about 125 µg per mL PCR liquid.

In an embodiment, the first compartment is configured to have a temperature in a range of about 85°C to about 105°C, preferably about 98°C.

In an embodiment, the second compartment is configured to have a temperature in a range of about 45°C to about 72°C, preferably about 60°C to about 72°C.

In an embodiment, the optional third compartment is configured to have a temperature in a range of about 65°C to about 75°C, preferably about 72°C.

Merely as an example, the tube may have a total length of about 15 m, comprising about 25 helical stacks (or cycles) of tube windings. In that specific embodiment, in one helical stacks (or cycle) the tube may be guided through a first compartment (100cm; temperature of about 85°C to about 105°C) followed by a second compartment (100cm; temperature of about 45°C to about 72°C) followed by a third compartment (400cm; temperature of about 65°C to about 75°C). The dwell time in each compartment may be adjusted by the flow rate of the PCR and the diameter of the tube.

The person of skill in the art understands that different DNA products (e.g. different size and/or sequence) may require different PCR conditions (e.g. due to DNA primer annealing temperatures or elongation times etc.). By adjusting the length of the tube, the length of the tube in the different compartments, the number of helical stacks (and thus the number of PCR cycles), the flow rate, the diameter of the tube, and/or the temperatures in the compartments, different PCR programs suitably for various different DNA products can be realized. Accordingly, the device for preparing a DNA product by means of Capillary Polymerase Chain Reaction can easily be adapted and adjusted to allow for the preparative production of DNA products of various sizes and sequences.

In **a second aspect,** the present invention is directed to a manufacturing device for a pharmaceutical product comprising the device for preparing a DNA product by means of Capillary Polymerase Chain Reaction of the first aspect.

In an embodiment, the manufacturing device for a pharmaceutical product comprises the device for preparing a DNA product by means of Capillary Polymerase Chain Reaction as described in the first aspect and at least one further functionality (e.g. additional chambers, additional units, a housing, etc.). In a preferred embodiment, the manufacturing device for a pharmaceutical product according to the second aspect comprises a process chamber, a technical chamber and the device for preparing a DNA product by means of Capillary Polymerase Chain Reaction as described in the first aspect.

### Description of the manufacturing device and preferred embodiments thereof

The manufacturing device for a pharmaceutical product can generally be understood as a closed housing with different chambers for different purposes.

The process chamber is suitable and used for manufacturing the pharmaceutical product. The process chamber may also be referred to as "wet part" of the manufacturing device in a sense that essentially all wet processes during the production process take place in the process chamber. "Wet processes" means e.g. the provision of buffers and/or reactants and/or products to and from vessels or to and from chromatographic columns, the collection of waste fluid or waste gases, etc., as well as any connections of such buffers and/or reactions and/or products (e.g. connections in the form of flexible tubes). Such wet processes should ideally be separated from any technical media supply (in particular electric supply such as power cables) in order to render impossible or at least minimize accidents due to leakage of liquids in the process chamber and possible consequences thereof, in particular due to voltage. Should accidents due to leakage occur in the process chamber, safety is improved as there is the afore-mentioned separation, and it will only be required to clean the process chamber. The device according to the first aspect is located in the process chamber.

The technical chamber is suitable and used for providing technical support e.g. for housing apparatus, as e.g. a pump, a motor, a mixer, a processor or the like, which are not in direct contact with the process media. The technical chamber is typically suitable and used for providing (e.g. housing) technical media supply, which is in particular power supply, e.g. a power supply for an apparatus/unit that is located in the process chamber and that is in direct contact with the process media. The technical chamber may also be referred to as "dry part" of the manufacturing device in a sense that essentially no wet processes as defined above take place in the technical chamber. As noted above, this *inter alia* results in increased safety. One may refer to the technical chamber as being "not as clean" as the process chamber since the required level of cleanliness in the technical chamber is lower than in the process chamber. The technical media supply for the device according to the first aspect is located in the technical chamber.

The chambers may be separated by a separation element, which is plate-shaped, in particular plate-shaped when looking at the separation element from the process chamber. Thus, as will be outlined below, in order not to disturb the gas flow, the separation element may comprise at least one appendix with an opening towards the process chamber and an extension in the direction of the technical chamber, where apparatus may be housed that are in contact with the process media, in particular the device according to the first aspect.

The manufacturing device comprising the device for preparing a DNA product by means of Capillary Polymerase Chain Reaction preferably further comprises a control unit configured to control a gas flow through the process chamber as a gas shower. The gas flow may be provided by a flow unit. The flow unit may be at least a pump or a HVAC system, and may also be referred to as fan-system. The flow unit may be arranged outside the housing of the manufacturing device (external flow unit) or preferably within the housing of the manufacturing device (internal flow unit). The flow unit preferably comprises a filter unit (which may be referred to as "fan filter unit" and which preferably comprises a HEPA filter as described below). The flow unit may be configured to provide, preferably variable, volumes of gas. The gas may be clean air, air with additives, an inert gas or any other gas. The control unit can be understood as any control device for a gas flow, as e.g. a valve or a processor. The control unit may be configured to adjust different pressures in the gas flow. The control unit may be configured to control the gas flow through the process chamber to provide a positive pressure in the process chamber. The positive pressure in the process chamber can be understood as overpressure relative to the environment outside the housing and/or a higher pressure than in the technical chamber. The positive pressure in the process chamber may protect open process steps from particulate contamination.

The control unit is configured to control the gas flow through the process chamber to provide the gas shower through the process chamber. The gas shower may fall from a higher level in the process chamber to a lower level in the process chamber. The gas shower in the process chamber may protect open process steps from particulate contamination. In an embodiment, the gas shower is laminar, which can be understood as essentially similar to a laminar flow or essentially unidirectional or as essentially not turbulent. The laminar gas shower or gas flow may be directed downwardly or in horizontal direction, preferably in a constant stream, towards a bottom of the process chamber. The laminar gas shower may allow reducing and maintaining extremely low levels of particulates, such as dust, airborne organisms, or vaporized particles, without introducing turbulences and/or contaminating e.g. a manufacturing procedure in the respective housing or chamber. Apparatus mounted in the area of the gas shower may be mounted tightly to the separation element or a process chamber wall or be covered or encapsulated to reduce or eliminate a turbulence or disturbance of the gas shower. Apparatus may also be "hidden" in an appendix of the separation element or in in an appendix of a process chamber wall to reduce or eliminate a turbulence or disturbance of the gas shower. In an embodiment, the speed of the gas shower is in a range of about 0.2 to about 0.6 m/s, preferably about 0.36 to about 0.54 m/s. This speed may provide a good balance between reducing and maintaining extremely low levels of particulates and not disturbing e.g. a manufacturing procedure in the process chamber.

The manufacturing device for a pharmaceutical product according to the second aspect allows for a flexible adaption to different conditions, requirements and e.g. quantities of pharmaceutical product as the process is operated in a continuous or semi-continuous mode. The present manufacturing device allows for a preparation of large quantities of a pharmaceutical product. Further, the manufacturing of a pharmaceutical product is generally scalable.

The present manufacturing device may provide a closed (bio-synthetic) process from raw materials (e.g. from dNTPs, DNA primers, DNA template) to a final product. The present manufacturing device can be understood as manufacturing-in-a-box. The present manufacturing device can be dimensioned to be very small. It may have a low footprint and form a low footprint-manufacturing entity. The footprint may be in a range of about 100 x about 100 x about 200 cm such that it can easily e.g. shipped and stored in a typical shipping container of the usual size. The present manufacturing device is thus portable to allow transportation to e.g. regions of an outbreak of a pandemic. It thereby generally allows a local, decentral production of a pharmaceutical product (e.g. a DNA vaccine, DNA template).

The present manufacturing device generally allows for a very fast manufacturing of pharmaceutical products. A change and preparation of the manufacturing device to a specific pharmaceutical product as well as the manufacturing of the specific pharmaceutical product itself may be very fast. There may be no need for adjustment or calibration steps. There may be no or fewer need for cleaning steps. The turnover time for e.g. a vaccine manufacturing may be less than about a week.

The present manufacturing device is generally very easy to clean, which allows reducing the cleaning efforts. The reason is a sanitary and hygienic design right from the beginning. It may comprise an aluminium housing, wherein the aluminium may have been pre-treated with e.g. an anodic oxidation and passivation, an anti-microbial surface finish with e.g. silver ion containing colours, electro polished surfaces, a use of compatible materials throughout the manufacturing device, accessibility of many or every part of the manufacturing device for cleaning, smooth, seamless and cleanable exposed surfaces, harmonized inner diameters and geometries for optimal automated cleaning in place (CIP) procedures and/or anti-microbiological cleansing, a sealing of any crevices or hollow areas, a reduction or stop of any build-up of condensation and precipitation, etc. In particular, the housing, the chamber(s) and/or the separation element may be made of aluminium, preferably surface-treated aluminium to allow for CIP. Additionally or alternatively, surfaces outside and/or within the housing and in particular within the chamber(s) may have an anti-microbial surface finish. Further, apparatus within the housing and in particular within the process chamber may be encapsulated to provide a smooth surface. Further, moving parts or parts directing technical media may be encapsulated to be safe and to allow an easy cleaning. Additionally or alternatively, apparatus within the housing and in particular within the process chamber may be embedded into a wall and in particular into the separation element to be, in best case, flush with the respective wall or surface. These options improve the cleanability as well as maintain the gas flow without disruptions or undesired circumventions due to non-flush elements in the process chamber.

The present manufacturing device can be operated under GMP (guidelines for good manufacturing practice) - compliant conditions. At least the process chamber, preferably the manufacturing device, is operable according to the requirements of the EU guidelines for good manufacturing practice for medicinal products, annex 1. In an embodiment, the GMP requirements are the requirements of the EU Guidelines to Good Manufacturing Practice Medicinal Products for Human and Veterinary Use, Annex 1, Manufacture of Sterile Medicinal Products (corrected version), European Commission, Brussels, 25 November 2008 (revised). In another embodiment, the GMP requirements are the requirements of the FDA (2004) Guidance for Industry, Sterile Drug Products Produced by Aseptic Processing - Current Good Manufacturing Practice, U.S. Department of Health and Human Services, Food and Drug Administration, Center for Drug Evaluation and Research (CDER) Center for Biologics Evaluation and Research (CBER) Office of Regulatory Affairs (ORA) Pharmaceutical CGMPs. In another embodiment, the present manufacturing device may be operated compliant to CE, ISO or GAMP5 Standards. The present manufacturing device may allow a GMP compliant manufacturing-in-a-box. GMP compliant conditions may comprise compliant machine controls and data logging, as for example automatic logging and reporting of critical process parameters and/or quality attributes, e.g. pressure, humidity, temperature, UV-absorption, total organic carbon, infrared absorption spectra, flow, power consumption, an error causing event (opened door, leakage, shortcut, pressure loss...) and the like.

In an embodiment, the manufacturing device comprising a device for preparing a DNA product by means of Capillary Polymerase Chain Reaction further comprises a separation element separating the process chamber from the technical chamber. The process chamber may be suitable and used for a manufacturing of a pharmaceutical product. The technical chamber may be suitable and used for housing apparatus, as e.g. a pump, a motor, a mixer, a processor or the like. The separation element is plate shaped. The separation element may be used to mount apparatus thereto. Mounted apparatus may be covered to reduce a turbulence or disturbance of the gas flow, prevent precipitation and/or ease a cleaning. The separation element may extend (only) between the process chamber and the technical chamber or it may extend at least partially or completely through the housing. The separation element may also extend outside the housing to separate parts of the surrounding environment, as e.g. a surrounding cleanroom.

In an embodiment, the separation element comprises an appendix with an opening towards the process chamber. The appendix may protrude in the direction of the technical chamber and may have an extension in the direction of the technical chamber, which serves as an extended process room portion. The separation element and the appendix can be understood as a plate with a blind hole. The appendix may be used to house an apparatus at least partially or fully. The device for preparing a DNA product (which may alternatively be referred to as "PCR reactor") may be insertable into the appendix. The device for preparing a DNA product or PCR reactor may therefore comprise and be housed in a box to ease the insertion into the appendix. The apparatus as e.g. the PCR reactor may then have no or only minimal intrusion into the process chamber but can still be regarded as being located in the process chamber. This allows increasing the available space in the process chamber without increasing a length of the process chamber. Further, it may allow a plug & play of different apparatus for e.g. different or new PCR processes (e.g. different PCR reactors reflecting different PCR programs e.g. by having different tube lengths).

In an embodiment, at least a handle for handling the device for preparing a DNA product in and/or out of the appendix is positioned at the front face of the device for preparing a DNA product to ease a handling of the device for preparing a DNA product.

In an embodiment, the appendix comprises an exchange rail system to guide and ease an exchange of the device for preparing a DNA product with another device. The exchange rail system may comprise exchange rails arranged at the appendix or at the device for preparing a DNA product and in particular at its box. The device for preparing a DNA product may further comprise a handling device as e.g. a trolley, lift or the like to allow and/or ease an exchange of apparatus from e.g. the process chamber side.

In an embodiment, a front face of the device for preparing a DNA product or of its box ends essentially flush with the opening of the appendix into the process chamber when the device for preparing a DNA product is inserted into the appendix. As a result, the gas flow and in particular the gas shower may be not or less disturbed and/or precipitation surfaces are reduced.

In an embodiment, the intake for a PCR liquid and/or an outfall for a DNA product is/are positioned at the front face of the device for preparing a DNA product or of its box. In another embodiment, the intake for a thermal medium and/or an outfall for the thermal medium is/are positioned at a back plate of the device for preparing a DNA product opposite to the front face of the device for preparing a DNA product.

In another, less preferred embodiment, vice versa, the appendix may also have an opening towards the technical chamber and an extension in the direction of the process chamber. In all cases, the appendix may be closable by a cover.

In an embodiment, the manufacturing device comprising the device for preparing a DNA product by means of Capillary Polymerase Chain Reaction further comprises a leakage sensor arranged in the appendix outside the device for preparing a DNA product. The leakage sensor may increase the safety of the device for preparing a DNA product. As noted above in the first aspect, the manufacturing device may also comprise the second part of a proximity sensor, wherein the first part is located at the device of the first aspect. Once the device has been installed properly in the manufacturing device, the two parts of the proximity sensor are connecting and preferably signaling that the installing was successful and/or preferably only then the device can successfully be started and operated. Accordingly, the proximity sensor comprising the two parts may also be active during operation and result in an alarm and/or stop of the operation once the two parts of the proximity sensor lost their connection.

In an embodiment, the pump unit for pumping the PCR liquid through a tube of the device for preparing a DNA product is arranged outside the device of the first aspect. It is preferred that the pumping unit is arranged in the process chamber of the manufacturing device for a pharmaceutical product. Alternatively, the pumping unit may be an external pumping unit. The external arrangement may save space within the manufacturing device and/or may allow a use of a powerful pumping unit. The pumping unit may be a pump.

In an embodiment, the manufacturing device comprising the device for preparing a DNA product by means of Capillary Polymerase Chain Reaction further comprises a coupling unit arranged at the casing of the manufacturing device for a pharmaceutical product and configured to couple the manufacturing device for a pharmaceutical product to another manufacturing device. The coupling unit may allow and ease a coupling of two or more manufacturing devices. A resulting manufacturing module is described herein in the third aspect.

The manufacturing device for a pharmaceutical product can be understood as a closed housing with a technical chamber and a process chamber. In an embodiment, the housing is sealed relative to the environment. In the same or another embodiment, the process chamber is sealed relative to the technical chamber. Throughout this application, the term "sealed" can be understood as compliant with at least IP64. IP codes are based on Degrees of protection provided by enclosures (IP Code) (IEC 60529:1989 + A1:1999 + A2:2013); German version EN 60529:1991 + A1:2000 + A2:2013 and/or ISO 20653:2013-02 Road vehicles - Degrees of protection (IP code) - Protection of electrical equipment against foreign objects, water and access. The term "sealed" can be understood as dust-tight and protection against splashing of water. "Dust-tight" can be understood as no ingress of dust and complete protection against contact. For testing, a vacuum is typically applied, test duration of up to 8 hours based on airflow, details can be found in above cited standards. "Protection against splashing of water" can be understood as water splashing against an enclosure from any direction shall have no harmful effect, utilizing either an oscillating fixture for 10 minutes or a spray nozzle with no shield for 5 minutes minimum, details can be found in above cited standards. A sealed housing or a sealed process chamber may have the benefit to be in particular clean, which means to have and to maintain low levels of particulates, such as dust, airborne organisms, or vaporized particles.

In an embodiment, the process chamber is a clean room and/or is configured to be at least a grade D room according to the above mentioned EU guidelines for good manufacturing practice for medicinal products, annex 1. Preferably, the process chamber is configured to be at least a grade C room. More preferably, the process chamber is configured to be at least a grade B room. Even more preferably, the process chamber is configured to be a grade A room. Grade A to D rooms may provide the benefit and are designed to be in particular clean, which means to have and to maintain extremely low levels of particulates, such as dust, airborne organisms, or vaporized particles. Cleanliness may be quantified by a number of particles per cubic meter at a predetermined molecule measure. A characterization of the A to D grades can be found in below inserted table 1. The process chamber can thereby be classified according to a number and size of particles permitted per volume of gas. The number and size of particles can be measured by particle counting either based upon light scattering, light obscuration, or direct imaging. A high intensity light source is used to illuminate the particle as it passes through a detection chamber. The particle passes through the light source (typically a laser or halogen light) and if light scattering is used, then the redirected light is detected by a photo detector. If direct imaging is used, a halogen light illuminates particles from the back within a cell while a high definition, high magnification camera records passing particles. The recorded video is then analysed by computer software to measure particle attributes. If light blocking (obscuration) is used, a loss of light is detected. The amplitude of the light scattered or light blocked is measured and the particle is counted and tabulated into standardized counting bins. Direct imaging particle counting employs a use of a high resolution camera and a light to detect particles. Vision based particle sizing units obtain two-dimensional images that are analysed by computer software to obtain particle size measurement.

**Table 1: EU GMPs Annex 1 Recommended Limits for Particulate Contamination**

| | **Maximum permitted number of particles per m³ equal to or greater than the tabulated size** | | | |
|---|---|---|---|---|
| | **At rest** | | **In operation** | |
| **Grade** | **0.5 µm** | **5.0 µm** | **0.5 µm** | **5.0 µm** |
| **A** | **3,520** | **20** | **3,520** | **20** |
| **B** | **3,520** | **29** | **352,000** | **2,900** |
| **C** | **352,000** | **2,900** | **3,520,000** | **29,000** |
| **D** | **3,520,000** | **29,000** | **Not defined** | **Not defined** |

A comparison of the A to D grade room according to EU guidelines for good manufacturing practice for medicinal products, annex 1, and the FED-STD-209e (1992) Federal Standard 209-e Airborned Particulate Cleanliness Classes in Cleanrooms and Clean Zones, the ISO (1999) International Standard 14644-1: Cleanrooms and associated controlled environments-Part 1: Certification of Air Cleanliness. International Organisation for Standardisation, Switzerland, as well as the ISO (2003) International Standard ISO 14698-2:2003 Cleanrooms and associated controlled environments - Biocontamination control -Part 2: Evaluation and interpretation of biocontamination data is shown in below inserted Table 2.

**Table 2: Comparison of classification systems**

| • WHO Technical Report Series, No. 902,2002 Annex 6 | | | | |
|---|---|---|---|---|
| Comparison of different airborne particulate classification systems for clean areas^{a} | | | | |
| WHO (GMP) | United States (209E) | United States (customary) | ISO/TC (209) | EEC (GMP) |
| Grade A | M 3.5 | Class 100 | ISO 5 | Grade A |
| Grade B | M 3.5 | Class 100 | ISO 5 | Grade B |
| Grade C | M 5.5 | Class 10000 | ISO 7 | Grade C |
| Grade D | M 6.5 | Class 100000 | ISO 8 | Grade D |
| EEC: European Commission; ISO/TC: International Organization for Standardization Technical Committee. | | | | |

In an embodiment, the control unit is configured to control the gas flow to provide a gas flow from the process chamber into the technical chamber. This can be understood in that the gas flows from the process chamber to and into the technical chamber. In the same or another embodiment, both chambers are arranged so that the gas flow in the technical chamber is parallel to the gas flow in the process chamber, but in an opposite direction. Preferably, there is the following gas flow within the housing: to the process chamber, into the process chamber, through the process chamber, out of the process chamber, to the technical chamber, into the technical chamber, through the technical chamber, and out of the technical chamber, while not all steps are necessary.

The gas may enter the housing before entering the process chamber. The gas may exit the housing after the technical chamber. Therefore, the manufacturing device may further comprise at least a duct through the housing for a passage of the gas flow between the housing and the environment. The duct may be an inlet and/or an outlet for the gas. Preferably, the duct is sealed relative to the environment. As stated above, the term "sealed" can be understood as dust-tight and protected against splashing of water as well as compliant with at least IP64. The seal might be a silicone seal.

In an embodiment, the housing comprises a passage for the gas flow from the process chamber to the technical chamber. The passage can be understood as a channel for the gas. The passage or channel may be provided as a duct or as an intermediate chamber arranged outside the process chamber and the technical chamber and (downstream) between the process chamber and the technical chamber. The passage or channel may also transvers the separation element as a sealed conduit or sealed through hole. As stated above, the term "sealed" can be understood as dust-tight and protected against splashing of water as well as compliant with at least IP64. The sealed conduit may have the benefit to maintain low levels of particulates. The sealed conduit may also comprise a filter to further reduce a level of particulates and/or a valve unit to control the gas flow. The valve unit may be a valve, preferably a throttle valve.

In an embodiment, the control unit is configured to control the gas flow to provide a pressure difference between the process chamber and the environment in a range of about 5 to about 100 Pa, preferably about 10 to about 20 or to about 15 Pa. The pressure difference may preferably be at least about 15 Pa. This can be understood in that there is a higher pressure in the process chamber than in the environment, including the technical chamber. At least the pressure in the process chamber can be higher than atmosphere pressure. The higher pressure in the process chamber makes it almost impossible for any particles to enter and contaminate the process chamber. In an embodiment, the control unit is configured to control the gas flow to provide a pressure difference between the technical chamber and the environment in a range of about -5 to about -100 Pa, preferably about -10 to about -20 Pa. The pressure difference may preferably be at least about -15 Pa. In an embodiment, the control unit is configured to control the gas flow to provide a pressure difference between the process chamber and the technical chamber in a range of about 10 to about 200 Pa, preferably about 20 to about 40 Pa. The pressure difference may preferably be at least about 30 Pa.

In an embodiment, there is a higher pressure in the process chamber than in the technical chamber. This is fulfilled if either the pressure in the process chamber and the pressure in the technical chamber are higher than atmosphere pressure or the pressure in the process chamber is higher than atmosphere pressure and the pressure in the technical chamber is the same as atmosphere pressure or the pressure in the process chamber is higher than atmosphere pressure and the pressure in the technical chamber is below atmosphere pressure. In other words, in an embodiment, the control unit is configured to control the gas flow to provide in the technical chamber a negative pressure relative to the environment.

In an embodiment, the manufacturing device for a pharmaceutical product further comprises a fluid collect tray to collect fluid leakages. The tray can be arranged at a bottom of the housing and/or in the passage from the process chamber to the technical chamber. This is beneficial as humidity can be collected before harming the product or the apparatus arranged in the chamber. Humidity can be intentionally implemented or can derive from the gas expansion in the technical chamber.

In an embodiment, the manufacturing device for a pharmaceutical product further comprises a sensor unit comprising at least one of a group of a flow sensor, a pressure sensor, a temperature sensor, a humidity sensor and a leakage sensor. The sensor unit may be arranged in the housing, e.g. at the separation element, in the process chamber, in the technical chamber, in the intermediate chambers there between or the intermediate chamber as part of the technical chamber, in the conduits trough the separation element, at the bottom of the housing etc. The sensor unit may allow controlling the conditions in view of e.g. flow rate, pressure, temperature, humidity, leakage etc. Accordingly, the sensor unit may comprise at least one of a group of a flow sensor, a pressure sensor, a temperature sensor, a humidity sensor, a microbial sensor, a particulate sensor, an organics sensor, and a leakage sensor.

In an embodiment, the manufacturing device for a pharmaceutical product further comprises a coupling unit arranged at an outer wall of the housing and configured to couple the manufacturing device to another manufacturing device. The other manufacturing device may be similar to the above described manufacturing device for a pharmaceutical product or it might be a different device. The coupling unit may allow a mechanical coupling of at least two manufacturing devices. The coupling unit may allow a fluid communication and coupling of at least two manufacturing devices. The coupling unit may allow a data communication and coupling of at least two manufacturing devices. The fluid communication includes in particular the transport of a pharmaceutical product from one manufacturing device to another manufacturing device, for example the transport of a DNA template after its purification and/or filtration in a first manufacturing device from this first manufacturing device to a second manufacturing device, wherein the DNA template may then be used in an RNA generation unit comprised in a second manufacturing device.

It is preferred that the coupling unit is also arranged to comprise a quality control unit, wherein this quality control unit is suitable for analyzing samples that are taken and/or provided from the communication, preferably the fluid communication between the devices. Thus, as exemplified above, if e.g. a DNA template is transported from a first device to a second device, a sample may be taken and analyzed by the quality control unit, which is preferably located in or at the coupling unit.

The pharmaceutical product can be understood as an active pharmaceutical ingredient (e.g. a DNA vaccine) or any precursor or any intermediate thereof (e.g. a DNA template for RNA in vitro transcription). Thus, the pharmaceutical product may be the active pharmaceutical ingredient that is used in a medicament and administered to e.g. a human subject in order to treat or prevent a disease, or an intermediate thereof.

### Production of a DNA-based pharmaceutical product

It may be desirable to produce a DNA-based pharmaceutical product, such as in particular a DNA vaccine (e.g. a lipid nanoparticle encapsulated DNA vaccine). In essence, the production of the DNA vaccine can be initiated once the sequence of the target is known, e.g. the sequence of a viral protein (e.g. the spike protein in case of SARS-CoV2). The process then typically follows the steps of i) de-novo synthesis of the DNA with the desired sequence; ii) DNA amplification by a device according to the first aspect of the present application, optionally followed by a DNA modification; iii) encapsulation of the DNA into the formulation; and iv) filling and finishing the product, wherein each step builds upon the previous step. Of course, typical purification and filtration step will also be carried out in between the afore-mentioned steps, if applicable.

In this embodiment, the manufacturing device for a pharmaceutical product according to the present application further comprises at least one of a group comprising a process media supply unit, a mixing unit, a de-novo DNA synthesis unit, a formulation unit, a purification unit, a filtration unit, a DNA modification unit, a fill-and-finish unit, and combinations thereof. The required units may be present in either one manufacturing device or in at least two manufacturing devices that are coupled resulting in a manufacturing module as described herein in the third aspect. In an especially preferred embodiment, each unit comprises a technical media supply, wherein the technical media supply is located in the technical chamber, a process media supply and/or a device configured to be in contact with the process media, wherein the process media supply and the device are located in the process chamber, and a sealed through hole located in the separation element and configured to connect the technical media supply and the device configured to be in contact with the process media. A unit may furthermore comprise a waste outlet, where e.g. wash buffer or by-products are collected.

In a preferred embodiment, the manufacturing device for a pharmaceutical product comprising a device according to the first aspect further comprises a process media supply unit, a mixing unit, a purification unit, an optional DNA modification unit, and a filtration unit, wherein the units are preferably connected in the following order i) a process media supply unit, ii) a mixing unit, iii) the device according to the first aspect, optionally followed by a DNA modification unit, iv) a purification unit and v) a filtration unit, and the device is configured to amplify DNA, wherein the amplified DNA is optionally modified. The DNA template for the device according to the first aspect can be generated as de-novo-synthesized DNA in another manufacturing device that is connected to the manufacturing device according to the present embodiment. The amplified DNA may be used in a separate manufacturing device that is connected to the device of this embodiment and that comprises a DNA formulation unit.

### Production of an RNA-based pharmaceutical product

It may be desirable to produce an RNA-based pharmaceutical product, such as in particular an RNA vaccine (e.g. a lipid nanoparticle encapsulated RNA vaccine). In essence, the production of the RNA vaccine can be initiated once the sequence of the target is known, e.g. the sequence of a viral protein (e.g. the spike protein in case of SARS-CoV2). The process then typically follows the steps of i) de-novo synthesis of the DNA with the desired sequence; ii) DNA template generation by a device according to the first aspect of the present application, optionally followed by a DNA modification; iii) RNA generation; iv) encapsulation of the RNA into the formulation; and v) filling and finishing the product, wherein each step builds upon the previous step. Of course, typical purification and filtration step will also be carried out in between the afore-mentioned steps, if applicable.

In this embodiment, the manufacturing device for a pharmaceutical product according to the present application further comprises at least one of a group comprising a process media supply unit, a mixing unit, a de-novo DNA synthesis unit, a DNA modification unit, an RNA generation unit, a formulation unit, a purification unit, a filtration unit, a fill-and-finish unit, and combinations thereof. The required units may be present in either one manufacturing device or in at least two manufacturing devices that are coupled resulting in a manufacturing module as described herein in the third aspect. In an especially preferred embodiment, each unit comprises a technical media supply, wherein the technical media supply is located in the technical chamber, a process media supply and/or a device configured to be in contact with the process media, wherein the process media supply and the device are located in the process chamber, and a sealed through hole located in the separation element and configured to connect the technical media supply and the device configured to be in contact with the process media. A unit may furthermore comprise a waste outlet, where e.g. wash buffer or by-products are collected.

In a preferred embodiment, the manufacturing device for a pharmaceutical product comprising a device according to the first aspect further comprises a process media supply unit, a mixing unit, a purification unit, an optional DNA modification unit and a filtration unit, wherein the units are preferably connected in the following order i) a process media supply unit, ii) a mixing unit, iii) the device according to the first aspect, optionally followed by a DNA modification unit, iv) a purification unit and v) a filtration unit, and the device is configured to produce template DNA, wherein the amplified DNA is optionally modified. This template DNA may be used in a separate manufacturing device that is connected to the device of this embodiment and that comprises an RNA generation unit.

The units disclosed above are described in further detail in the third aspect below.

For both, the production of a DNA-based pharmaceutical product and the production of an RNA-based pharmaceutical product, the device according to the first aspect of the invention (which is comprised in the manufacturing device according to the second aspect) will of course be suitable for amplification of the particular DNA to be amplified, in particular by having the tube wound through the compartments and stacked such that the resulting PCR conditions and cycles are optimal for this particular DNA to be amplified. If the amplification of a second, different DNA should become necessary, the first device or PCR reactor can easily be replaced in the manufacturing device by a different device or PCR reactor according to the first aspect, which is suitable for amplification of the second DNA to be amplified, in particular by having the tube wound through the compartments and stacked such that the resulting PCR conditions and cycles are optimal for this second DNA to be amplified. Accordingly, the manufacturing device can easily be equipped (or "loaded") with the PCR reactor suitable for the amplification of the specific DNA of interest, and this easy handling and adaption, respectively, may be referred to as "plug and play" system.

In **a third aspect,** the present invention is directed to a manufacturing module for a pharmaceutical product. The manufacturing module comprises at least two manufacturing devices including a device according to the second aspect. The at least two manufacturing devices are coupled with each other. The coupling may allow a mechanical coupling of the at least two manufacturing devices. The coupling may allow a fluid communication and coupling of the at least two manufacturing devices. The coupling may allow a data communication and coupling of the at least two manufacturing devices. The fluid communication includes in particular the transport of a pharmaceutical product from one device to another device, for example the transport of a DNA template after its purification and/or filtration in a first device from this first device to a second device, wherein the DNA template may then be used in an RNA generation unit comprised in the second device.

The manufacturing module may in particular be for the production of DNA template and then comprise a process media supply unit, a mixing unit, a device according to the first aspect (which may also be referred to as "DNA template generation unit"), an optional DNA modification unit, a purification unit and a filtration unit, wherein the units are preferably connected in the order as listed and the module is configured to produce template DNA. The amplification process (or "DNA template generation process") is carried out using the device according to the first aspect.

The manufacturing module comprises a manufacturing device for a pharmaceutical product according to the second aspect and may further comprise at least one manufacturing device selected from the group consisting of a manufacturing device comprising a process media supply unit and a mixing unit, a manufacturing device comprising a purification unit, and a manufacturing device comprising a filtration unit.

In one embodiment, the manufacturing module comprises (i) a first manufacturing device comprising a process media supply unit and a mixing unit, (ii) a second manufacturing device according to the second aspect, (iii) a third manufacturing device comprising a purification unit and (iv) a fourth manufacturing device comprising a filtration unit, wherein the devices are preferably connected in the order as listed. Such a manufacturing module is configured to produce template DNA, preferably from DNA (optionally de-novo synthesized DNA) that is provided in the form of process media supply in the PCR liquid. In other words, such a manufacturing module is configured to produce from a small amount of DNA, which may be de-novo synthesized DNA and which may be produced by a preceding manufacturing module, a large amount of template DNA. This template DNA may optionally be modified by a DNA modification unit comprised in yet another manufacturing device.

This template DNA can in particular be used when producing RNA, preferably mRNA, by RNA in vitro transcription. Accordingly, either in a combined or in a separate manufacturing module, an RNA generation unit (for transcribing the obtained DNA into RNA) may be comprised in.

In one embodiment, the manufacturing module comprises a process media supply unit, a mixing unit, a de-novo DNA synthesis unit, a device according to the first aspect (which may be referred to as "DNA template generation unit"), optionally a DNA modification unit, a purification unit, a filtration unit, a formulation unit and an RNA generation unit, wherein the units are preferably arranged in the order of i) a process media supply unit, ii) a mixing unit, iii) a de-novo DNA synthesis unit, iv) a purification unit, v) a filtration unit, vi) a process media supply unit, vii) a mixing unit, viii) a device according to the first aspect, optionally in combination with a DNA modification unit, ix) a purification unit, x) a filtration unit, xi) a process media supply unit, xii) a mixing unit, xiii) an RNA generation unit, xiv) a purification unit, xv) a filtration unit, xvi) a process media supply unit, xvii) a formulation unit, xviii) a purification unit and xix) a filtration unit, and the module is preferably configured to produce formulated RNA, preferably formulated mRNA, more preferably LNP-formulated mRNA.

In an embodiment, the manufacturing module for a pharmaceutical product comprises i) a manufacturing device comprising a process media supply unit and a mixing unit, ii) a manufacturing device comprising a de-novo DNA synthesis unit, iii) a manufacturing device comprising a purification unit, iv) a manufacturing device comprising a filtration unit, v) a manufacturing device comprising a process media supply unit and a mixing unit, vi) a manufacturing device according to the second aspect, optionally in combination with a DNA modification unit, vii) a manufacturing device comprising a purification unit, viii) a manufacturing device comprising a filtration unit, ix) a manufacturing device comprising a process media supply unit and a mixing unit, x) a manufacturing device comprising a bioreactor for RNA in vitro transcription, xi) a manufacturing device comprising a purification unit, xii) a manufacturing device comprising a filtration unit, xiii) a manufacturing device comprising a process media supply unit and a mixing unit, xiv) a manufacturing device comprising a filtration unit, preferably a tangential flow filtration unit, and xv) a manufacturing device comprising a filtration unit, preferably a sterile filter, wherein the devices are coupled in the order as given. This module is preferably configured to produce formulated RNA, preferably formulated mRNA, more preferably LNP-formulated mRNA.

The present manufacturing module for a pharmaceutical product provides a very flexible and versatile platform. It can be adapted to various purposes and uses. It allows for a modular manufacturing or production of pharmaceutical products. It may be understood as an entity suitable for modular manufacturing, which means that it can be adapted to various purposes. An entity can be sufficient for a specific manufacturing method or several entities can be connected to perform a specific manufacturing method, e.g. in the form of a manufacturing line or chain. Such modular design allows easy handling, process improvements and/or maintenance procedures. The technologies and operations used within the present manufacturing module as well as the choice of product-contacting materials can be pre-evaluated before using to provide a high quality manufacturing of pharmaceutical products. As a result, the present manufacturing module allows a very robust and reproducible manufacturing of the pharmaceutical product. Further, the present manufacturing module generally allows a (fully or partially) automated manufacturing of a pharmaceutical product.

In an embodiment, the process media supply unit comprises a mounting element configured to hold a process medium supply container in the process chamber. Typically, there are several mounting elements, each configured to hold a process medium supply container, which is then connected, e.g. via sterile tubes, to the downstream unit or device, such as e.g. the DNA de-novo synthesis unit, the DNA template generation unit, the RNA generation unit or the formulation unit. The downstream unit or device may also be a purification unit, such as e.g. a reversed-phase HPLC column. The process medium can be selected from the group comprising synthetic DNA for amplification in a PCR reaction, a PCR component mix, water, in-vitro-translation buffer, a nucleotide including UTP, GTP, ATP and CTP, an enzyme including an RNA polymerase, a buffer including a wash buffer, a reagent buffer (e.g. an immobilization buffer) and an elution buffer, HPLC buffers, a formulation solution including a lipid solution, and an HPLC eluent.

In an embodiment, the mixing unit comprises a mixer. It may also comprise a pump unit (e.g. a syringe pump, pulseless flow pump, peristaltic pump or the like). The mixing unit may e.g. be used in combination with either the DNA template generation unit or the RNA generation unit, where the mixing unit is present upstream of either the DNA template generation unit or the RNA generation unit and provides respective mastermixes for the subsequent DNA template generation unit or the RNA generation unit. A mixing unit may also be used when a polyvalent pharmaceutical product is produced, such as e.g. a polyvalent RNA-based pharmaceutical product that comprises at least two different RNA sequences. In the case of such polyvalent RNA-based pharmaceutical products, a mixing unit may be used to mix the at least two different RNAs (i.e. the at least two RNAs with different sequences) prior to these RNAs being formulated in the formulation unit. Alternatively, at least two different RNAs that are already formulated may be mixed by a mixing unit in order to obtain a polyvalent RNA-based pharmaceutical product.

The mixing unit may also be used when formulating the nucleic acid product, in particular the RNA, and may in this case be referred to as formulation unit. Thus, the mixing unit may be used for formulation when lipid nanoparticle (LNP) or liposome encapsulated RNA or RNA complexed with a polycationic peptide or protein (e.g. protamine or a polymeric carrier, e.g. a polyethylene glycol/peptide polymer e.g. according to WO2012/013326) is generated. In the context of formulation, the mixing unit may comprise at least two pump units (e.g. one unit for pumping the lipids, one unit for pumping the RNA), and, optionally, a reactor for complexation/formulation (e.g. a T-piece connector).

In an embodiment, the de-novo DNA synthesis unit comprises a solid-phase synthesis unit for DNA, typically using the phosphoramidite method and corresponding phosphoramidite building blocks derived from protected 2'-deoxynucleosides in order to obtain DNA-oligonucleotides of about 200 nucleotides in length. Preferably in a fully automated manner, the phosphoramidite building blocks are sequentially coupled according to the sequence that is to be produced (which may also be referred to as in silico designed sequence), wherein the oligonucleotide-product is typically released from the solid phase to solution, then deprotected and collected. The de-novo DNA synthesis unit may furthermore be configured to assemble (or couple) at least two oligonucleotides obtained by the phosphoramidite method in order to obtain longer sequences, if applicable. As noted above, a purification unit will typically follow the de-novo DNA synthesis unit. De-novo DNA synthesis may also be based on enzymatic processes e.g. based on terminal deoxynucleotidyl transferase (TdT) as described in WO2015159023. Additionally, the de-novo DNA synthesis unit may comprise a ligation unit for ligating the de-novo synthetized DNA into an appropriate DNA backbone.

In an embodiment, the DNA modification unit comprises a suitable means to either enzymatically or chemically modify the amplified DNA. An enzymatic modification may e.g. be a digestion reaction with a restriction endonuclease to cut the DNA at a specific position (e.g. to provide a DNA ending with a poly(A). A chemical modification may e.g. be the coupling of the 5' and/or 3' end to a tag or the like or the chemical modification of the 5' and/or 3' end. Such a DNA modification unit may be operable in a continuous mode, where e.g. the amplified PCR product resulting from the device according to the first aspect of the present application is fed into the DNA modification unit. If the DNA modification unit is configured for enzymatic DNA modification by restriction endonucleases, it is preferred that the respective restriction endonucleases is immobilized (e.g. as described in WO2016174227). A DNA modification unit as defined above may in some embodiments even be comprised in the device of the first aspect.

In an embodiment, the RNA generation unit comprises a bioreactor for RNA in vitro transcription, preferably a bioreactor as disclosed in WO 2020/002598. In particular, a bioreactor as defined by Claims 1 to 59 in WO 2020/002598, or as illustrated by Figures 1 to 14 in WO 2020/002598 may be used as an RNA generation unit. It is especially preferred for the bioreactor that the DNA used as template in the bioreactor is immobilized on magnetic particles and that the bioreactor comprises a magnet for mixing the reaction solution including the DNA immobilized on magnetic particles.

In an embodiment, the purification unit comprises an HPLC unit, preferably a unit for performing RP-HPLC. Particularly preferred in that context is RP-HPLC using a method disclosed in WO2008/077592 preferably using a porous, non-alkylated poly(stryrene-divinylbenzene) reverse phase, wherein the reverse phase is formed by beads or occurs as a polymerized block (e.g. monolithic). Alternatively, or in addition, the purification unit may comprise an affinity chromatography unit, preferably an oligo dT purification unit for affinity purification of polyadenylated nucleic acid, in particular RNA, via oligo dT functionalized matrices or beads or columns (e.g. as described in WO2014152031A1). Alternatively, or in addition, the purification unit may comprise an anion exchange chromatography unit. Alternatively, or in addition, the purification unit may comprise a unit for nucleic acid precipitation and a unit for purifying said precipitated nucleic acid (e.g. using TFF or centrifugation or filtration). Alternatively, or in addition, the purification unit may comprise a unit for dsRNA removal e.g. a unit that involves RNase III treatment or a unit that involves a step of cellulose-based dsRNA purification. The purification unit may be used to purify in particular a nucleic acid, preferably the template DNA obtained in the de-novo DNA synthesis unit, the DNA obtained in the device according to the first aspect and/or the RNA obtained in the RNA generation unit. In preferred embodiments, the purification unit, in particular the purification unit for purifying RNA comprises an RP-HPLC unit and an oligo dT purification unit and, optionally, a unit for dsRNA removal.

In an embodiment, the filtration unit comprises a tangential-flow filtration unit. Particularly preferred in that context is tangential flow filtration as described in WO2016/193206, wherein TFF is used for diafiltration and/or concentration and/or purification of a nucleic acid. The filtration unit may be used to filter in particular a nucleic acid, preferably the template DNA or the RNA, after the purification unit. It can also be preferred that the filtration unit comprises a sterile filter, preferably a sterile filter with a size of 0.22 µm, in order to sterile-filter in particular a formulation comprising the RNA, in particular mRNA, more particularly LNP-formulated mRNA.

In a preferred embodiment, the fill-and-finish unit comprises a filling and/or dosing machine. The fill-and-finish unit is configured to fill a formulated pharmaceutical product, in particular formulated active pharmaceutical ingredient (in particular the formulated RNA, more particularly the LNP-formulated mRNA) into suitable vials, such as glass vials in an amount that corresponds to a single dose or multiple doses of the formulated active pharmaceutical ingredient. Typically the vials, such as the glass vials, are closed with caps (e.g. aluminium caps) under sterile conditions. In embodiments, the fill-and-finish unit comprises a freezing unit for freezing the obtained drug product to at least -20°C, preferably to at least -60°C or -80°C.

In **a fourth aspect,** the present invention is directed to a method for preparing a DNA product by means of Capillary Polymerase Chain Reaction. The method for preparing a DNA product comprises the following steps, not necessarily in this order:
- providing a first compartment, a second compartment, and a tube, wherein the tube is wound at least from the first compartment to the second compartment and from the second compartment to the first compartment, wherein the tube wound from compartment to compartment forms a helical stack of tube windings,
- providing means to adjust a separate temperature in each compartment, and
- guiding a PCR liquid through the tube and thereby through the separate temperatures in each compartment to prepare the DNA product based on the PCR liquid.

The method according to the present invention for preparing a DNA product allows for a flexible adaption to different conditions, requirements and e.g. quantities of DNA. The present method allows for the preparation of large quantities of DNA (e.g. more than 2 mg per reaction, even up to g-amounts). Further, the preparation of a DNA product may be scalable. The present method for preparing a DNA product allows for a very fast preparation of a DNA product and thereby also a fast manufacturing of a pharmaceutical product. Also a change and adaption of the device for preparing a DNA product to a specific DNA product may be very fast. There may be no need for adjustment or calibration steps. There may be no or fewer need for cleaning steps. The turnover time for e.g. a vaccine manufacturing may be less than about a week.

In an embodiment, the temperature in the first compartment is configured for a denaturation step of the Polymerase Chain Reaction, the temperature in the second compartment is configured for an annealing step of the Polymerase Chain Reaction and optionally also for an elongation step of the Polymerase Chain Reaction.

In an embodiment, the temperature in the first compartment is in a range of about 85°C to about 105°C, preferably about 98°C. In an embodiment, the temperature in the second compartment is in a range of about 45°C to about 72°C, preferably about 60°C to about 72°C.

In an embodiment, the method comprises the provision of a third compartment. The tube is wound at least from the second compartment to the third compartment, and then back to the first compartment. The temperature in the third compartment is configured for an elongation step of the PCR, preferably in a range of about 65°C to about 75°C, preferably about 72°C.

In an embodiment, the method for preparing a DNA product is carried out under GMP-compliant conditions.

In an embodiment, the means to adjust a separate temperature in each compartment may be the provision of a separate thermal medium flow through each compartment by means of a thermal medium inlet and a thermal medium outlet in each compartment (to adjust a separate temperature in each compartment).

In another embodiment, the means to adjust a separate temperature in each compartment may be a heating unit (in particular a heating cartridge) and optionally a cooling unit (in particular a thermoelectric cooler) comprised in each compartment (optionally connected to a ventilator).

In **a fifth aspect,** the present invention is directed to the use of the device for preparing a DNA product according to the first aspect for preparing a DNA product by means of Capillary Polymerase Chain Reaction, the use of the manufacturing device for a pharmaceutical product product according to the second aspect for the production of a pharmaceutical product, and the use of the manufacturing module according to the third aspect for the production of a pharmaceutical product.

The pharmaceutical product may be formulated. The pharmaceutical product is preferably an active pharmaceutical ingredient in the form of a biomolecule or any precursor or any intermediate thereof, wherein the biomolecule is preferably a nucleic acid, more preferably DNA. The production is preferably GMP-compliant.

In an embodiment, the above uses are automated. This means the use or the operation of the device for preparing a DNA product by means of Capillary Polymerase Chain Reaction according to the first aspect, the manufacturing device for a pharmaceutical product according to the second aspect or the manufacturing module according to the third aspect can be fully or partially automated. This may allow a more reliable, faster and/or more cost effective manufacturing of a pharmaceutical product.

It should be noted that the aspects of the invention described above apply to the device for preparing a DNA product by means of Capillary Polymerase Chain Reaction; the manufacturing device for a pharmaceutical product; the manufacturing module for a pharmaceutical product; the method for preparing a DNA product by means of Capillary Polymerase Chain Reaction; the use of the device for preparing a DNA product by means of Polymerase Chain Reaction; and the use of the device for a production of a pharmaceutical product according to the independent claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

### BRIEF DESCRIPTION OF THE DRAWINGS

The Figures shown in the following are merely illustrative and shall describe the present invention in a further way. These figures shall not be construed to limit the present invention thereto.
- **Figure 1**: **shows a top view on a device according to the present invention for preparing a DNA product by means of Capillary Polymerase Chain Reaction.**
- **Figure 2**: **shows a side view on a device according to the present invention for preparing a DNA product by means of Capillary Polymerase Chain Reaction.**
- **Figure 3**: **shows a lateral 3D view of a wound tube.**
- **Figure 4**: **shows a lateral 3D view of a tube wound around several stands.**
- **Figure 5**: **shows a 3D view of a manufacturing device for a pharmaceutical product.**
- **Figure 6a**: **shows a 3D view of a box housing the device for preparing a DNA product to be inserted into the manufacturing device for a pharmaceutical product.**
- **Figure 6b**: **shows a 3D view of a box housing the device for preparing a DNA product to be inserted into the manufacturing device for a pharmaceutical product.**
- **Figure 7**: **shows schematically and exemplarily a manufacturing module for a pharmaceutical product according to the invention.**
- **Figure 8**: **shows a lateral 3D view on a device according to the present invention.**
- **Figure 9**: **shows a lateral 3D view on the top of a device according to the present invention.**
- **Figure 10**: **shows a lateral 3D view on the box housing the device to be inserted into a manufacturing device.**
- **Figure 11**: **shows a 3D view zooming into a manufacturing device with the box housing the device inserted into the manufacturing device.**

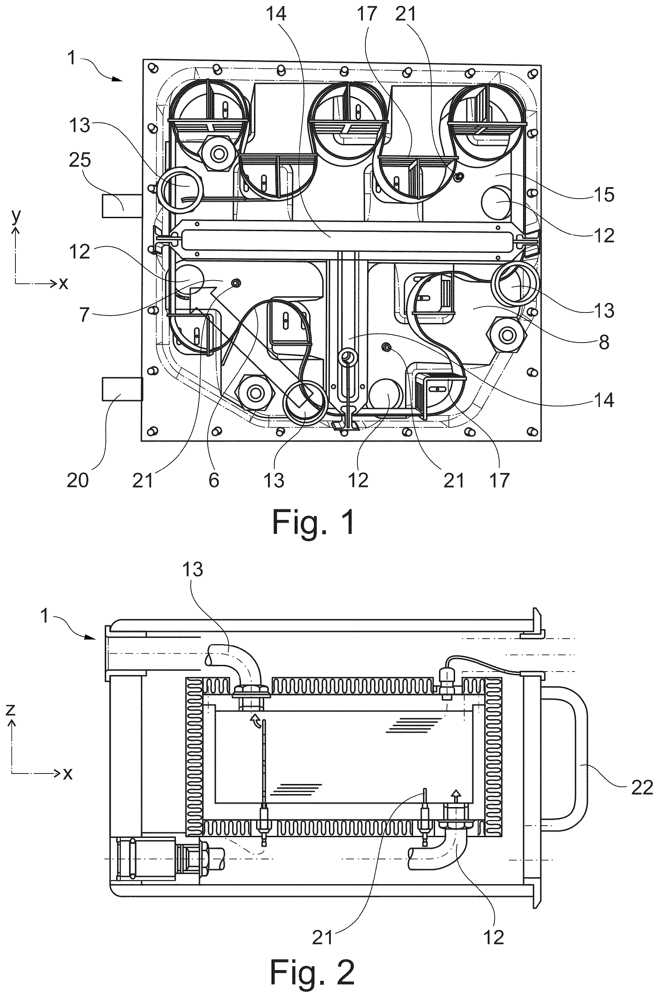

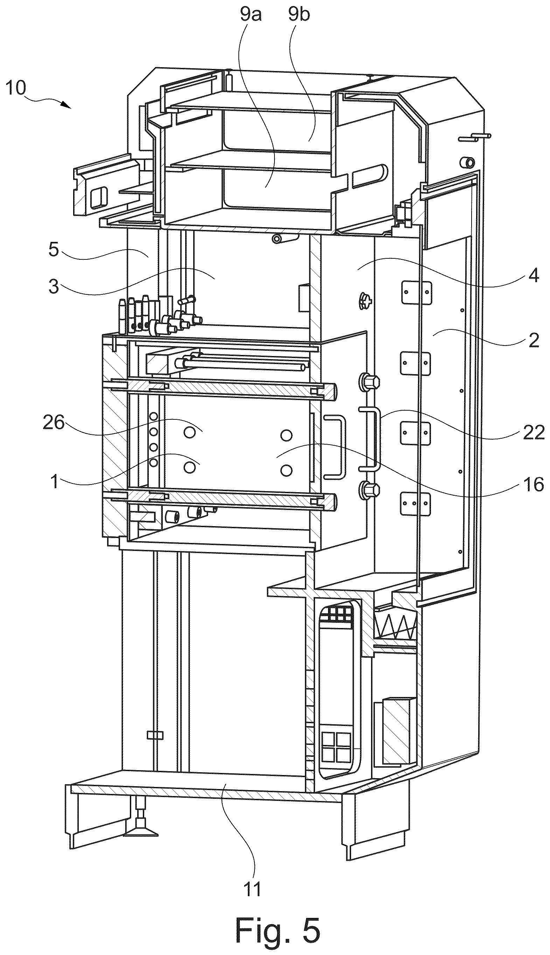

**Figure 1** shows a top view on a device 1 according to the present invention for preparing a DNA product by means of Capillary Polymerase Chain Reaction. **Figure 2** shows a side view of a device 1 according to the present invention for preparing a DNA product by means of Capillary Polymerase Chain Reaction. **Figure 8** shows a lateral 3D view on the device according to the present invention. In Figure 2, the device 1 for preparing a DNA product is mounted into a manufacturing device 10 according to the present invention for a pharmaceutical product, which will be explained in more detail in view of Figure 5. The DNA product can be DNA to be used as API or a DNA template for enzymatic RNA in vitro transcription, where the RNA would be the API. Polymerase Chain Reaction (PCR) is a technology to synthetically amplify DNA across several orders of magnitude, generating thousands to millions of copies of a particular DNA sequence. Capillary Polymerase Chain Reaction can be understood as PCR done in a capillary or tube 6. The present device 1 for preparing a DNA product allows for a flexible and fast preparation of large quantities of a DNA product. The device 1 for preparing a DNA product may be used for a flexible and fast vaccine production, particularly a DNA vaccine production or production of a DNA template for enzymatic RNA in vitro transcription. The present device 1 may be in particular used for preparing DNA templates for mRNA-based vaccines e.g. during infectious disease epidemics and pandemics.

As shown in Figure 1 and Figure 8, the device 1 for preparing a DNA product comprises a tube 6, a first compartment 7, a second compartment 8, and a third compartment 15. The tube 6 or capillary has a very small diameter, which can be in a range of about 0.5 mm to about 50 mm, preferably about 0.5 mm to about 1.5 mm, more preferably about 0.75 to about 1.25 mm. The tube 6 is wound from the first compartment 7 to the second compartment 8 and from the second compartment 8 to the third compartment 15. It then comes back to the first compartment 7. A PCR liquid can be filled into the tube 6 and thereby be guided through the three compartments. As shown in Figures 2 and 8, the compartments can be understood as independent basins, e.g. to be filled by a thermal medium (see Figure 2) or with a thermal solid (see Figure 8). The basins are here at least partially, preferably completely surrounded by a thermal insulation, which may be the septum wall 14 (see Figure 1) or the inner-compartment wall 30 (see Figure 8), optionally in combination with the septum wall 14.

The tube 6 is wound to form a helical stack of tube windings, which can be understood as a helix, spiral or coil of winding when seen in a lateral view. **Figure 3** shows a lateral 3D view of a wound tube 6. There can be several layers of windings, e.g. 30. The helical extension or stacking direction of the tube 6 extends in a z-direction essentially perpendicular to an x, y plane formed by a bottom of the device 1 for preparing a DNA product or a plane formed by the floor.

Back to Figures 1 and 8, the tube 6 is not only wound from the first compartment 7 to the second compartment 8 and from the second compartment 8 to the third compartment 15, but also further from the third compartment 15 to the first compartment 7, from the first compartment 7 to the second compartment 8, and from the second compartment 8 to the third compartment 15, etc. Each tube winding represents a PCR cycle.

The tube 6 extends within the three compartments in a corrugated manner, which can be understood in that the tube 6 has a shape of a wave or a snake within all three compartments when seen in a top view. The corrugation of the tube 6 extends in x- and y-directions of the plane formed by the bottom of the device 1 for preparing a DNA product or the plane formed by the floor. The corrugated form of the tube 6 defines a dwell time of the PCR liquid in the respective compartment. The corrugation direction of the tube 6 is essentially perpendicular to the stacking direction of the tube 6.

As shown in Figure 1, the device 1 for preparing a DNA product further comprises a PCR liquid inlet 20 for a PCR mix, which is connectable to an external pump unit or pump (37) for pumping the PCR liquid through the tube 6. The device 1 for preparing a DNA product further comprises a DNA product outlet 25 for a PCR liquid product. Alternatively, the PCR device may comprise an internal pump unit or pump (37) for pumping the PCR liquid through the tube 6. As shown in Figure 1, the PCR liquid inlet 20 may be located at the first compartment, whereas the DNA product outlet 25 may be located at the third compartment.

As shown in Figures 1 and 2, the device 1 for preparing a DNA product in this embodiment further comprises a thermal medium inlet 12 and a thermal medium outlet 13 in each of the three compartments. As noted above, it may comprise other means to adjust the temperature in each compartment. If thermal medium is used, the thermal medium may enter each of the three compartments by the respective thermal medium inlet 12 and may exit each of the three compartments by the respective thermal medium outlet 13. This may lead to a flow of thermal medium through the respective compartment. The medium outlet 13 is preferably positioned at the top to facilitate the removal of gas bubbles.

The thermal medium can be understood as a liquid heat carrier and can be used to provide three different temperature zones in the three compartments to prepare the DNA product by thermal cycling. The three temperatures zones correspond to the temperature profiles of the PCR for denaturation, annealing, and elongation. The PCR liquid is separated by the tube walls from the thermal medium in the compartments. The tube walls allow a temperature exchange between the PCR liquid and the inside of the compartments, e.g. the thermal medium, which can be used to heat or cool the PCR liquid, e.g. by means of the thermal medium.

The thermal medium inlet 12 and the thermal medium outlet 13 are in this embodiment arranged within each compartment to provide the thermal medium flow through the compartment essentially in a counter direction to a guiding direction of the PCR liquid in the tube 6. As shown in Figure 1, this can be understood that in case of a guiding direction of the PCR liquid from top left to bottom right, the thermal medium inlet 12 and the thermal medium outlet 13 are arranged to provide the thermal medium flow in a counter direction from bottom right to top left (see the arrow in the first compartment 7). The counter flow of thermal medium and PCR liquid improves a temperature exchange between the thermal medium and the PCR liquid.

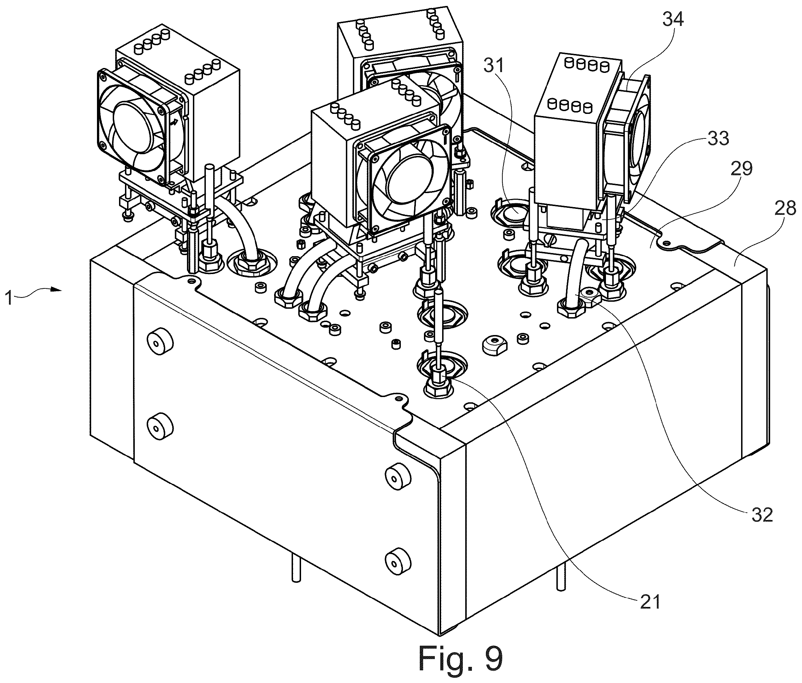

As shown in **Figure 8****,** the device 1 in this embodiment comprises an isolation 28 and a shell 29 as well as an inner-compartment wall 30 that surrounds the tube in a corrugated matter in each of the compartments 7, 8 and 15. Each compartment comprises at least one heating unit 31 (here depicted as heating cartridge) as well as a cooling unit, here comprising a heat pipe 32, which can be used for cooling, if necessary. In this embodiment, the space in each compartment surrounded by the inner-compartment wall 30 (wherein this space surrounds the tube 6) is filled with a thermal solid (such as e.g. aluminum-pellets), optionally in combination with a liquid or gel, wherein the thermal solid is heated by the at least one heating unit 31 as well as - if necessary - cooled by a cooling unit (comprising heat pipe 32) in order to provide a specific temperature in each compartment. The space between the compartments and/or the surrounding shell 29, i.e. the space that does not surround the tube 6, can be filled with synthetic material (e.g. synthetic balls or granules). **Figure 9** shows the top view of the device 1 shown in Figure 8, wherein the isolation 28 and the shell 29 are depicted, and also the top of the heating unit 31 as well as the heat pipe 32. The heat pipe 32 is connected to a thermoelectric cooler 33 (e.g. a copper-block with a Peltier element) and a ventilator 34 to transport the heat away. Also in this embodiment, three different temperature zones are provided in the three compartments to prepare the DNA product by thermal cycling. The three temperatures zones correspond to the temperature profiles of the PCR for denaturation, annealing, and elongation. The PCR liquid is separated by the tube walls from the thermal solid in the compartments.

As shown in Figures 1 and 8, the device 1 for preparing a DNA product further comprises septum walls 14 between the compartments to insulate the compartments from each other (in Figure 8, the septum walls 14 are present only partially as the inner-compartment walls 30 already separate the compartments from each other). One septum wall 14 is arranged between the first compartment 7 and the second camber, another septum wall 14 is arranged between the second camber compartment and the third camber and a further septum wall 14 is arranged between the third camber compartment and the first camber. As a consequence, the adjacent compartments are thermally insulated from each other and therefore different temperatures can be established and maintained in the adjacent compartments. A septum wall 14 can be a plate shaped element. The septum walls 14 between the compartments can be implemented by several walls or by only one (e.g. T-shaped or star shaped) wall. The septum walls 14 comprise through holes for the tube 6 to extend from one compartment to the next.

As shown in Figures 1 and 9, the device 1 for preparing a DNA product further comprises sensors 21, here e.g. temperature sensors 21, arranged in each compartment. It is preferred that several of these temperature sensors are present in each compartment.

The device 1 for preparing a DNA product further comprises stands 17 for holding the tube 6. **Figure 4** shows a lateral 3D view of a tube 6 wound around several stands 17. The stands 17 are also shown in Figures 1 and 8. The stands 17 are arranged in each compartment to hold and guide the tube 6. The stands 17 provide a fixation point as well as a deviation point for the corrugation of the tube 6. The stands 17 may therefore comprise slits or the like to mount the tube 6 (winding) to the stand 17. The tube 6 extends partially around an outer dimension of the stand 17. The stands 17 here have a T-shape or an L-shape, when seen in a top view, but also other shapes are possible, e.g. a cylindrical shape. The stands 17 may be exchangeable and repositionable in the compartment. The tube 6 is here the same throughout the device 1 for preparing a DNA product and may be exchangeable.

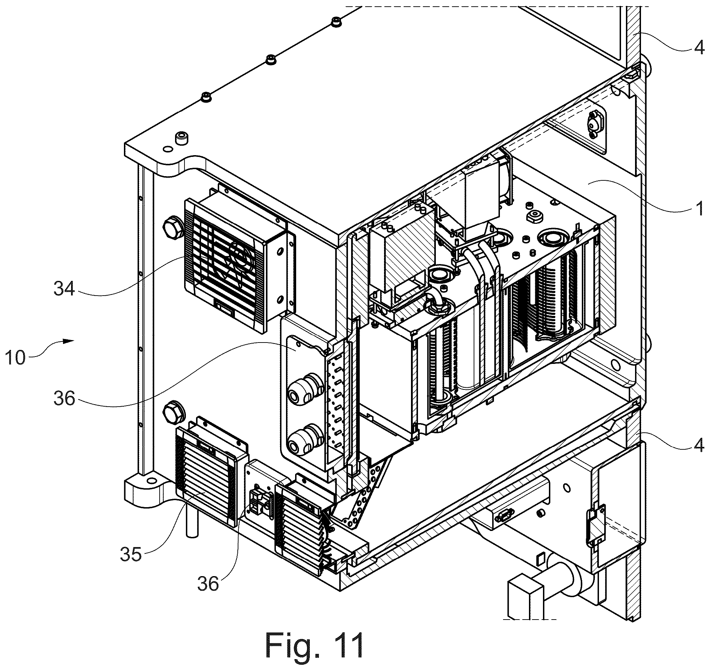

**Figure 5** shows a 3D view of a manufacturing device 10 for a pharmaceutical product. **Figure 11** shows a cut-out thereof. The manufacturing device 10 for a pharmaceutical product comprises a housing 5, a process chamber 2, a technical chamber 3 and a device 1 for preparing a DNA product by means of Capillary Polymerase Chain Reaction as described above. The pharmaceutical product can be understood as an active pharmaceutical ingredient or any precursor or any intermediate thereof (e.g. a DNA vaccine or a DNA template for RNA synthesis). The manufacturing device 10 for a pharmaceutical product can be operated under GMP (guidelines for good manufacturing practice)-compliant conditions.

The manufacturing device 10 for a pharmaceutical product can be understood as a closed and sealed housing 5 encompassing different chambers for different purposes. The process chamber 2 is suitable and used for manufacturing the pharmaceutical product. The process chamber 2 is here a grade A room. The process chamber 2 is sealed relative to the technical chamber 3. The technical chamber 3 is suitable and used for housing apparatus, as e.g. a pump, a motor, a mixer, a processor or the like. The chambers are separated by a separation element 4, which can be understood as a plate. The separation element 4 extends through the housing 5 and can be used to mount apparatus thereto.

The technical chamber 3 and the process chamber 2 are arranged so that a gas flow in the technical chamber 3 is parallel to a gas flow in the process chamber 2, but in an opposite direction. The gas of the gas flow is here clean air. There is a gas inlet duct 9a and a gas outlet duct 9b.

At least one control unit controls the gas flow through the process chamber 2 to provide a gas shower falling in the direction of gravity (downwards) and/or a positive pressure in the process chamber 2. The control unit can be a valve or a processor. The gas shower can be laminar. The gas flow can be provided by a flow unit, which may be arranged within or outside the housing 5 and may communicate with the control unit. The flow unit may be a pump, a HVAC system or the like. The positive pressure is an overpressure relative to the environment outside the housing 5. The flow unit provides variable volumes of gas, while the control unit adjusts different pressures in the gas flow.

As shown in Figure 5, the separation element 4 comprises at least an appendix 16 with an opening towards the process chamber 2 and an extension (extended process chamber) in the direction of the technical chamber 3. The appendix 16 is used to house apparatuses, as e.g. the above described device 1 for preparing a DNA product (which may also be referred to as "PCR reactor"). This allows increasing the available space in the process chamber 2. The device 1 for preparing a DNA product is here housed in a box 26, which is inserted as a unitary element into the appendix 16. The box 26 may be exchangeable.

As also shown in Figure 5, a front face of the device 1 for preparing a DNA product and in particular a front face of the box 26 ends essentially flush with the opening of the appendix 16 into the process chamber 2 when the device 1 for preparing a DNA product is inserted into the appendix 16. As a result, the gas flow and in particular the gas shower is seamless and may be not or less disturbed and/or precipitation surfaces are reduced. The appendix 16 and here its front face is closed by a cover. The cover may be part of the box 26 of the device 1 for preparing a DNA product

As shown in Figure 5, the manufacturing device 10 for a pharmaceutical product further comprises a fluid collect tray 11 to collect fluid leakages. The fluid collect tray 11 can be arranged at a bottom of the housing 5 and/or in the passage from the process chamber 2 to the technical chamber 3. The fluid collect tray 11 can be equipped with a leakage sensor 21.

Figure 11 shows the back of the manufacturing device 10 where the device 1 is inserted. At the back, which faces the technical chamber 3, a ventilator 34 is present. Further, an air inlet 35 is present, along with electronic plugs 36 to connect the device 1 to technical media, in particular power, in the technical chamber. This embodiment relates in particular to devices where a thermal solid is used for adjusting the temperature of the compartments (e.g. as exemplified in Figures 8, 9, and 10).

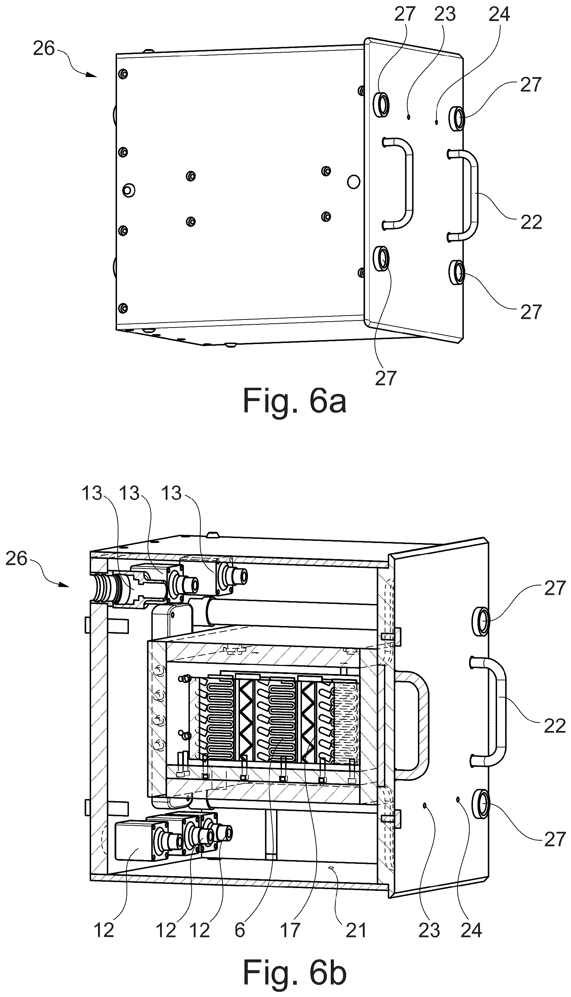

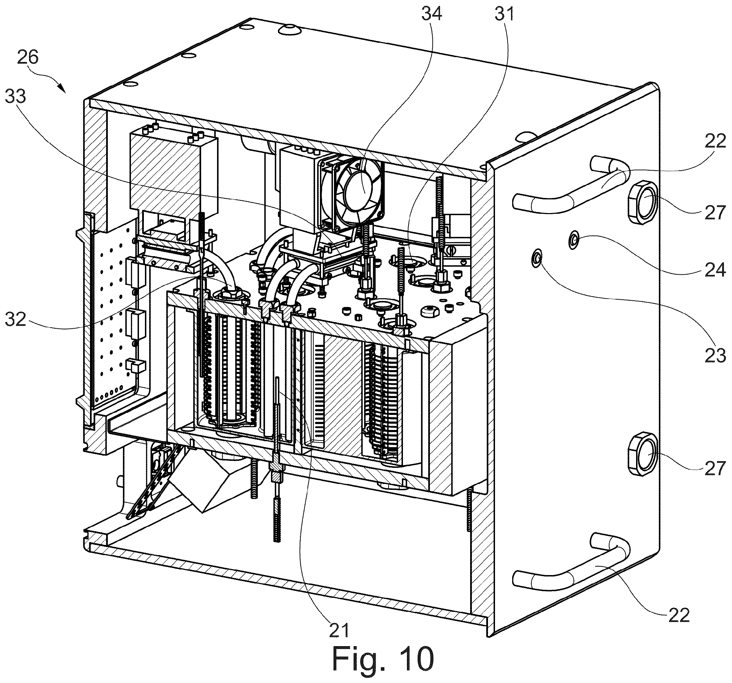

**Figures 6a and 6b** **as well as** **Figure 10** show 3D views of the box 26 housing the device 1 for preparing a DNA product to be inserted into the manufacturing device 10 for a pharmaceutical product. The box 26 is closed by the cover. The cover comprises handles 22 for handling the device 1 for preparing a DNA product in and/or out of the appendix 16. The box 26 comprises inlets 12 and outlets 13 for the thermal media for each compartment of the device 1 for preparing a DNA product (see Figure 6b) or heating cartridges 31 as well as heat pipes 32 connected to a thermoelectric cooler 33 and a ventilator 34 (see Figure 10). An intake for a PCR liquid 23 and/or an outfall for a DNA product 24 is/are positioned at the front face and here at the cover of the box 26 of the device 1 for preparing a DNA product. The appendix 16 (see Figure 5) and the box 26 comprise corresponding parts of an exchange rail system 27 to guide and ease an exchange of the apparatus in the appendix 16, as e.g. exchange the device 1 for preparing a DNA product by another device. As shown in Figure 6b and Figure 10, a sensor 21, e.g. a leakage sensor 21 or a temperature sensor 21, is arranged in the box 26. The box 26 further comprises connections to be connected to the process chamber 2, e.g. electric connections. In the embodiment according to Figure 10, the sensor is preferably a temperature sensor as the embodiment of Figure 10 (where a thermal solid is used as heating means) typically does not comprise a leakage sensor (as no thermal medium is used in the embodiment shown in Figure 10).

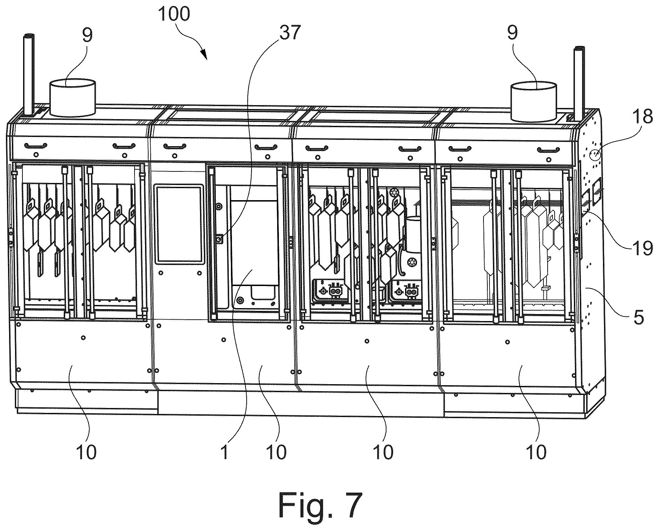

An external pump unit or pump (37 in Figure 7) for pumping the PCR liquid through the tube 6 of the device 1 for preparing a DNA product may be arranged outside the device 1, and may e.g. be located in the process chamber of the manufacturing device 10 for a pharmaceutical product.

**Figure 7** shows schematically and exemplarily a manufacturing module 100 for a pharmaceutical product according to the invention. The manufacturing module 100 comprises here four manufacturing devices 10. The manufacturing devices are very flexible and several manufacturing devices can be adapted to several manufacturing steps to form e.g. a manufacturing chain.

The four manufacturing devices 10 are coupled with each other by means of a coupling unit 18 arranged at an outer wall of the housing 5. The coupling unit 18 allows a fluid communication and coupling, and preferably also a mechanical coupling as well as a data communication and coupling of the manufacturing devices 10. The four manufacturing devices 10 are exemplary shown herein to be (from the left) a first device comprising a process media supply unit and a mixing unit, a second device comprising a manufacturing device 10 for a pharmaceutical product as described above (a device 1 for preparing a DNA product by means of Capillary Polymerase Chain Reaction ), a third device comprising a purification unit and a fourth device comprising a filtration unit, wherein the filtration unit comprises two filtration units, namely a tangential flow filtration unit and a sterile filter. This exemplary module can be used for the production of DNA and the amplification as well as purification of DNA, respectively. The exemplary module used for the production/amplification of DNA may be connected to further manufacturing devices that transcribe the obtained DNA into RNA (e.g. in a manufacturing device comprising an RNA bioreactor as described in WO2020002598).

The manufacturing module 100 comprises an attachment element 19 arranged at an outer wall of the housing 5 of the manufacturing device 10 outside the housing 5. The attachment element 19 is configured to hold a media supply (not shown) e.g. in form of a media reservoir.

### DEFINITIONS

For the sake of clarity and readability the following definitions are provided. Any technical feature mentioned for these definitions may be read on each and every embodiment of the invention. Additional definitions and explanations may be specifically provided in the context of these embodiments.

As used in the specification and the claims, the singular forms of "a" and "an" also include the corresponding plurals unless the context clearly dictates otherwise.

The term "about" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to encompass a group which preferably consists of these embodiments only.

The term "pharmaceutical product" as used herein relates to an active pharmaceutical ingredient or any precursor or any intermediate thereof. Thus, a "pharmaceutical product" may in particular be the active pharmaceutical ingredient that is used in a medicament and administered to a human or animal subject in order to treat or prevent a disease, i.e. it has a clinical grade, particularly when it comes to parameters such as purity, integrity. Such products are typically produced *in vitro* in a synthetic process, and the process of production includes precursors and intermediates. For the present invention, it is particularly preferred that the active pharmaceutical ingredient is a biomolecule, in particular a nucleic acid. The nucleic acid may be DNA. The DNA may particularly be used as a vaccine or as a DNA template for RNA in vitro transcription. When producing e.g. an mRNA as the active pharmaceutical ingredient, a first step may be the production of template DNA (also referred to as "a DNA template" herein), wherein this template DNA corresponds to a precursor of the mRNA since it serves as template in the *in vitro* transcription reaction when producing the RNA. An intermediate of the DNA production may be the DNA that is obtained from the *PCR* reaction before purification since such a DNA does not correspond to the final active pharmaceutical ingredient but *inter alia* requires that purification steps are carried out in order to provide the product in the required clinical grade.

The term "DNA" is the usual abbreviation for deoxyribonucleic acid. It is a nucleic acid molecule, i.e. a polymer consisting of nucleotide monomers. These nucleotides are usually deoxy-adenosine-monophosphate, deoxythymidine-monophosphate, deoxy-guanosine-monophosphate and deoxy-cytidine-monophosphate monomers or analogs thereof which are - by themselves - composed of a sugar moiety (deoxyribose), a base moiety and a phosphate moiety, and polymerize by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single stranded or double stranded. In the double stranded form, the nucleotides of the first strand typically hybridize with the nucleotides of the second strand, e.g. by A/T-base-pairing and G/C-base-pairing.

The term "RNA" is the usual abbreviation for ribonucleic acid. It is a nucleic acid molecule, i.e. a polymer consisting of nucleotide monomers. These nucleotides are usually adenosine-monophosphate (AMP), uridine-monophosphate (UMP), guanosine-monophosphate (GMP) and cytidine-monophosphate (CMP) monomers or analogs thereof, which are connected to each other along a so-called backbone. The backbone is formed by phosphodiester bonds between the sugar, i.e. ribose, of a first and a phosphate moiety of a second, adjacent monomer. The specific order of the monomers, i.e. the order of the bases linked to the sugar/phosphate-backbone, is called the RNA sequence. RNA can be obtained by transcription of a DNA sequence, e.g., inside a cell. In eukaryotic cells, transcription is typically performed inside the nucleus or the mitochondria. *In vivo,* transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, optionally a 5'UTR, a coding sequence, optionally a 3'UTR and a poly(A) sequence. If RNA molecules are of synthetic origin, as in the present invention, the RNA molecules are meant not to be produced *in vivo,* i.e. inside a cell or purified from a cell, but in an *in vitro* method. An examples for a suitable *in vitro* method is *in vitro* transcription. In addition to messenger RNA, several non-coding types of RNA exist which may be involved in regulation of transcription and/or translation, and immunostimulation and which may also be produced by *in vitro* transcription. The term "RNA" further encompasses RNA molecules, such as viral RNA, retroviral RNA and replicon RNA, small interfering RNA (siRNA), antisense RNA, saRNA (small activating RNA ), CRISPR RNA (small guide RNA, sgRNA), ribozymes, aptamers, riboswitches, immunostimulating RNA, transfer RNA (tRNA), ribosomal RNA (rRNA), small nuclear RNA (snRNA), small nucleolar RNA (snoRNA), microRNA (miRNA), and Piwi-interacting RNA (piRNA).

The term "RNA in vitro transcription" relates to a process wherein RNA is synthesized in a cell-free system. RNA may be obtained by DNA-dependent RNA in vitro transcription of an appropriate DNA template, which may be a linearized plasmid DNA template or a PCR-amplified DNA template. The promoter for controlling RNA in vitro transcription can be any promoter for any DNA-dependent RNA polymerase. Particular examples of DNA-dependent RNA polymerases are the T7, T3, SP6, or Syn5 RNA polymerases.

Reagents used in RNA in vitro transcription typically include: a DNA template (linearized DNA or linear PCR product) with a promoter sequence that has a high binding affinity for its respective RNA polymerase such as bacteriophage-encoded RNA polymerases (T7, T3, SP6, or Syn5); ribonucleotide triphosphates (NTPs) for the four bases (adenine, cytosine, guanine and uracil); optionally, a cap analogue (e.g. m7G(5')ppp(5')G (m7G) or a cap analogue derivable from the structure disclosed in claim 1-5 of WO2017/053297 or any cap structures derivable from the structure defined in claim 1 or claim 21 of WO2018075827); optionally, further modified nucleotides as defined herein; a DNA-dependent RNA polymerase capable of binding to the promoter sequence within the DNA template (e.g. T7, T3, SP6, or Syn5 RNA polymerase); optionally, a ribonuclease (RNase) inhibitor to inactivate any potentially contaminating RNase; optionally, a pyrophosphatase to degrade pyrophosphate (inhibitor of RNA synthesis); MgCl2, which supplies Mg2+ ions as a co-factor for the polymerase; a buffer (TRIS or HEPES) to maintain a suitable pH value, which can also contain antioxidants (e.g. DTT), and/or polyamines such as spermidine at optimal concentrations, e.g. a buffer system comprising Citrate and/or betaine as disclosed in WO2017/109161.

The nucleotide mixture used in RNA in vitro transcription may additionally contain modified nucleotides as defined herein. In that context, preferred modified nucleotides comprise pseudouridine (ψ), N1- methylpseudouridine (m1ψ), 5-methylcytosine, and 5-methoxyuridine. The nucleotide mixture (i.e. the fraction of each nucleotide in the mixture) used for RNA in vitro transcription reactions may be optimized for the given RNA sequence, preferably as described in WO2015188933.

An "RNA in vitro transcription (IVT) master mix" may comprise the components necessary for performing an RNA in vitro transcription reaction as defined above. Accordingly, an IVT master mix may comprise at least one of the components selected from a nucleotide mixture, a cap analogue, a DNA-dependent RNA polymerase, an RNAse inhibitor, a pyrophosphatase, MgCl2, a buffer, an antioxidant, betaine, Citrate.

As used herein, the term "template DNA" (or "a DNA template") typically relates to a DNA molecule comprising a nucleic acid sequence encoding the RNA sequence to be transcribed *in vitro.* The template DNA is used as template for RNA *in vitro* transcription in order to produce the RNA encoded by the template DNA. Therefore, the template DNA comprises all elements necessary for RNA *in vitro* transcription, particularly a promoter element for binding of a DNA dependent RNA polymerase as e.g. T3, T7 and SP6 RNA polymerases 5' of the DNA sequence encoding the target RNA sequence. Furthermore, the template DNA may comprise primer binding sites 5' and/or 3' of the DNA sequence encoding the target RNA sequence to determine the presence of the DNA sequence encoding the target RNA sequence e.g. by PCR or DNA sequencing.

The "polymerase chain reaction" or "PCR" is a technology in molecular biology used to amplify a piece of DNA across several orders of magnitude, generating thousands to millions of copies of a particular DNA sequence. The method relies on thermal cycling, consisting of cycles of repeated heating and cooling of the reaction for DNA melting and enzymatic replication of the DNA. Primers (short DNA fragments) containing sequences complementary to the target sequence along with a heat-stable DNA polymerase, such as Taq polymerase, enable selective and repeated amplification. As PCR progresses, the DNA generated is itself used as a template for replication, setting in motion a chain reaction in which the DNA template is exponentially amplified. The DNA polymerase enzymatically assembles a new DNA strand from DNA building-blocks, the nucleotides, by using single-stranded DNA as a PCR template and DNA oligonucleotides (also called DNA primers), which are required for initiation of DNA synthesis. The vast majority of PCR methods use thermal cycling, i.e., alternately heating and cooling the PCR sample through a defined series of temperature steps. In the first step, the two strands of the DNA double helix are physically separated at a high temperature in a process called DNA melting. In the second step, the temperature is lowered and the two DNA strands become templates for DNA polymerase to selectively amplify the target DNA. The selectivity of PCR results from the use of primers that are complementary to the DNA region targeted for amplification under specific thermal cycling conditions.

A "PCR master mix" or "PCR liquid" may comprise the components necessary for performing a PCR as defined above. Accordingly, a PCR master mix may comprise at least one of the components selected from a nucleotide mixture, a DNA polymerase, the synthetic DNA as (initial) template and a buffer.

The term "lipid nanoparticle" or "LNP" refers to a formulation of the pharmaceutical product, in particular the nucleic acid. In the context of the present invention, the term "LNP" is not restricted to any particular morphology, and includes any morphology generated when a cationic lipid and optionally one or more further lipids are combined, e.g. in an aqueous environment and/or in the presence of an nucleic acid. For example, a liposome, a lipid complex, a lipoplex and the like are within the scope of a lipid nanoparticle (LNP). LNPs typically comprise a cationic lipid and one or more excipients selected from neutral lipids, charged lipids, steroids and polymer conjugated lipids (e.g., PEGylated lipid). The nucleic acid may be encapsulated in the lipid portion of the LNP or an aqueous space enveloped by some or the entire lipid portion of the LNP. In one embodiment, the LNP consists essentially of (i) at least one cationic lipid; (ii) a neutral lipid; (iii) a sterol, e.g. cholesterol; and (iv) a PEG-lipid, e.g. PEG-DMG or PEG-cDMA, in a molar ratio of about 20-60% cationic lipid: 5-25% neutral lipid: 25-55% sterol; 0.5-15% PEG-lipid.

The term "process medium" refers to a component that is directly and physically involved in any reaction or any process step required to produce the pharmaceutical product. Accordingly, as the production is carried out in the process chamber, a "process medium" will be present in the process chamber when the device is used for production. A process medium can thus be in particular any starting material (such as e.g. a nucleotide), any catalyzing material (such as e.g. an enzyme) and any buffer (such as e.g. a reaction buffer or a purification buffer). A process medium is typically provided in a process medium supply container.

The term "technical medium" refers to a component that is not directly involved in any reaction or any process step required to produce the pharmaceutical product. Rather, a technical medium is indirectly involved, e.g. as a power cable that provides power to a unit or apparatus that processes the process medium, and will be located in the technical chamber. A technical medium supply will e.g. be power or power supply provided by a power cable.

It has to be noted that embodiments of the invention are described with reference to different subject matters. In particular, some embodiments are described with reference to method type claims whereas other embodiments are described with reference to the device type claims. However, a person skilled in the art will gather from the description that, unless otherwise notified, in addition to any combination of features belonging to one type of subject matter also any combination between features relating to different subject matters is considered to be disclosed with this application. However, all features can be combined providing synergetic effects that are more than the simple summation of the features.

If a group is defined to comprise at least a certain number of embodiments, this is also meant to encompass a group, which preferably consists of these embodiments only.

While the invention has been illustrated and described in detail in the drawings and the description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### List of references signs

- 1: device for preparing a DNA product
- 2: process chamber
- 3: technical chamber
- 4: separation element
- 5: housing
- 6: tube
- 7: first compartment
- 8: second compartment
- 9: duct
- 9a: gas inlet duct
- 9b: gas outlet duct
- 10: manufacturing device
- 11: collect tray
- 12: thermal medium inlet
- 13: thermal medium outlet
- 14: septum walls
- 15: third compartment
- 16: appendix
- 17: stand
- 18: coupling unit
- 19: attachment element
- 20: PCR liquid inlet
- 21: sensor
- 22: handle
- 23: intake for PCR liquid
- 24: outfall for a DNA product
- 25: DNA product outlet
- 26: box
- 27: exchange rails
- 28: isolation
- 29: shell
- 30: inner-compartment wall
- 31: heating unit
- 32: heat pipe (part of a cooling unit)
- 33: thermoelectric cooler (part of a cooling unit)
- 34: ventilator
- 35: air inlet
- 36: electric plug
- 37: pump unit for pumping the PCR liquid
- 100: manufacturing module

A set of embodiments of the present application relates to:
1. A device (1) for preparing a DNA product by means of Capillary Polymerase Chain Reaction (PCR), comprising:
   - a tube (6) for guiding a PCR liquid,
   - a first compartment (7), and
   - at least a second compartment (8),
   wherein the tube (6) is wound at least from the first compartment (7) to the second compartment (8), and wherein the first compartment (7) and the second compartment (8) each comprise a thermal medium inlet (12) and a thermal medium outlet (13) to provide a separate thermal medium flow through the first compartment (7) and the second compartment (8) to adjust a separate temperature in the first compartment (7) and the second compartment (8) to prepare the DNA product based on the PCR liquid.
2. Device (1) for preparing a DNA product according to embodiment 1, wherein septum walls (14) are arranged between the compartments to insulate the compartments from each other.
3. Device (1) for preparing a DNA product according to the preceding embodiment, wherein the septum walls (14) comprise through holes for the tube (6) to extend from one compartment to the next.
4. Device (1) for preparing a DNA product according to one of the preceding embodiments, wherein the thermal medium inlet (12) and the thermal medium outlet (13) are arranged within at least one and preferably within each compartment to provide the thermal medium flow through the compartment essentially in a counter direction to a guiding direction of the PCR liquid in the tube (6).
5. Device (1) for preparing a DNA product according to one of the preceding embodiments, wherein the thermal medium inlet (12) is arranged at a lower position in each of the compartments and the thermal medium outlet (13) is arranged at an upper position in each of the compartments.
6. Device (1) for preparing a DNA product according to one of the preceding embodiments, further comprising a third compartment (15), wherein the tube (6) is wound at least from the first compartment (7) to the second compartment (8) and from the second compartment (8) to the third compartment (15).
7. Device (1) for preparing a DNA product according to the preceding embodiment, wherein the tube (6) is further wound from the third compartment (15) to the first compartment (7), from the first compartment (7) to the second compartment (8), and from the second compartment (8) to the third compartment (15).
8. Device (1) for preparing a DNA product according to one of the preceding embodiments, wherein the tube (6) wound from compartment to compartment forms a helical stack of tube windings.
9. Device (1) for preparing a DNA product according to one of the preceding embodiments, wherein the tube (6) extends within at least one and preferably in each of the compartments in a corrugated manner.
10. Device (1) for preparing a DNA product according to embodiments 8 and 9, wherein a stacking direction of the helical stack of tube windings is different and preferably essentially perpendicular to a corrugation direction of the corrugated tube (6).
11. Device (1) for preparing a DNA product according to one of the preceding embodiments, wherein each compartment forms a basin to be filled by the thermal medium.
12. Device (1) for preparing a DNA product according to one of the preceding embodiments, further comprising at least a stand (17) arranged in one of the compartments to provide a fixation point for the tube (6) and/or a deviation point for a corrugation of the tube (6).
13. Device (1) for preparing a DNA product according to the preceding embodiment, wherein the tube (6) extends partially or fully or more than once around an outer dimension of the stand (17).
14. Device (1) for preparing a DNA product according to one of the embodiment 12 to 13, wherein the stand (17) is exchangeable and/or repositionable in the compartment.
15. Device (1) for preparing a DNA product according to one of the preceding embodiments, wherein the tube (6) comprises a PCR liquid inlet (20), which is connectable to a pump unit for pumping the PCR liquid through the tube (6).
16. Device (1) for preparing a DNA product according to the preceding embodiment, wherein the pump unit is arranged outside the compartments and preferably outside a shell (5) surrounding the compartments.
17. Device (1) for preparing a DNA product according to embodiment 15 or 16, wherein the pump unit is configured to provide a flow rate of the PCR liquid in a range of about 0.1 to about 10 mL/min, preferably about 0.2 to about 3 mL/min.
18. Device (1) for preparing a DNA product according to one of the preceding embodiments, wherein the tube (6) comprises a DNA product outlet (25) for a continuous preparation of the DNA product.
19. Device (1) for preparing a DNA product according to embodiment 1 to 17, wherein the tube (6) comprises a closable DNA product outlet (25) for a discontinuous preparation of the DNA product.
20. Device (1) for preparing a DNA product according to one of the preceding embodiments, further comprising at least a sensor (21) arranged in at least one of compartments, wherein the sensor (21) is configured to detect a temperature, a flow rate or a leakage of the compartment.
21. Device (1) for preparing a DNA product according to one of the preceding embodiments, wherein the tube (6) has a diameter in a range of about 0.5 to about 1.5 mm, preferably about 0.75 to about 1.25 mm.
22. Device (1) for preparing a DNA product according to one of the preceding embodiments, wherein the tube (6) has a length between a tube inlet and a tube outlet in a range of about 10 to about 200 m, preferably about 25 to about 50 m.
23. Device (1) for preparing a DNA product according to one of the preceding embodiments, wherein the tube (6) is dimensioned to contain PCR liquid in a range of about 10 mL to about 1L, preferably about 20 mL to about 250 mL.
24. Device (1) for preparing a DNA product according to one of the preceding embodiments, wherein the helical stack of tube windings comprises about 10 to about 50 windings, preferably about 15 to about 40.
25. Device (1) for preparing a DNA product according to one of the preceding embodiments, wherein the device (1) for preparing a DNA product is configured to prepare about 1 mg and more of the DNA product per reaction, preferably in a range of about 1 to about 30 mg, more preferably in a range of about 15 to about 25 mg.
26. Device (1) for preparing a DNA product according to one of the preceding embodiments, wherein the device (1) for preparing a DNA product is configured to prepare the DNA product in a range of about 50µg to about 150 µg per mL PCR liquid, preferably about 75 to about 125 µg per mL PCR liquid.
27. A manufacturing device (10) for a pharmaceutical product, comprising a process chamber (2), a technical chamber (3), and a device (1) for preparing a DNA product by means of Capillary Polymerase Chain Reaction according to one of the preceding embodiments.
28. Manufacturing device (10) for a pharmaceutical product according to the preceding embodiment, further comprising a control unit configured to control a gas flow through the process chamber (2) as a gas shower.
29. Manufacturing device (10) for a pharmaceutical product according to one of the embodiments 27 to 28, wherein at least the process chamber (2), preferably the manufacturing device (10), is operable according to the requirements of the EU guidelines for good manufacturing practice (GMP) for medicinal products, annex 1.
30. Manufacturing device (10) for a pharmaceutical product according to one of the embodiments 27 to 29, further comprising a separation element (4) separating the process chamber (2) from the technical chamber (3), wherein the separation element (4) comprises an appendix (16) with an opening towards the process chamber (2) and protruding in the direction of the technical chamber (3), and wherein the device (1) for preparing a DNA product is insertable into the appendix (16).
31. Manufacturing device (10) for a pharmaceutical product according to the preceding embodiment,
   wherein a front face of the device (1) for preparing a DNA product ends essentially flush with the opening of the appendix (16) into the process chamber (2) when the device (1) for preparing a DNA product is inserted into the appendix (16).
32. Manufacturing device (10) for a pharmaceutical product according to one of the embodiments 27 to 31, wherein at least a handle (22) for handling the device (1) for preparing a DNA product in and/or out of the appendix (16) is positioned at the front face of the device (1) for preparing a DNA product.
33. Manufacturing device (10) for a pharmaceutical product according to one of the embodiments 27 to 32, wherein an intake for a PCR liquid (23) and/or an outfall for a DNA product (24) is/are positioned at the front face of the device (1) for preparing a DNA product.
34. Manufacturing device (10) for a pharmaceutical product according to one of the embodiments 27 to 33, wherein an intake for a thermal medium and/or an outfall for the thermal medium is/are positioned at a back plate of the device (1) for preparing a DNA product opposite to the front face of the device (1) for preparing a DNA product.
35. Manufacturing device (10) for a pharmaceutical product according to one of the embodiments 27 to 34, further comprising a leakage sensor (21) arranged in the appendix (16) outside the device (1) for preparing a DNA product.
36. Manufacturing device (10) for a pharmaceutical product according to one of the embodiments 27 to 35, wherein the appendix (16) comprises exchange rails (27) to guide an exchange of the device (1) for preparing a DNA product with another device (1) for preparing a DNA product.
37. Manufacturing device (10) for a pharmaceutical product according to one of the embodiments 27 to 36, wherein a pump unit for pumping the PCR liquid through a tube (6) of the device (1) for preparing a DNA product is arranged outside a casing of the manufacturing device (10) for a pharmaceutical product.
38. Manufacturing device (10) for a pharmaceutical product according to one of the embodiments 27 to 37, further comprising a coupling unit (18) arranged at the casing of the manufacturing device (10) for a pharmaceutical product and configured to couple the manufacturing device (10) for a pharmaceutical product to another manufacturing device (10).
39. A manufacturing module (100) for a pharmaceutical product, comprising a manufacturing device (10) for a pharmaceutical product according to one of the embodiments 27 to 38 and at least one of a group comprising a manufacturing device (10) with a process media supply unit and a mixing unit, a manufacturing device (10) with a purification unit, and a manufacturing device (10) with a filtration unit.
40. A method for preparing a DNA product by means of Capillary Polymerase Chain Reaction (PCR), comprising:
   - providing a first compartment (7), a second compartment (8), and a tube (6), wherein the tube (6) is wound at least from the first compartment (7) to the second compartment (8),
   - providing a separate thermal medium flow through each compartment by means of a thermal medium inlet (12) and a thermal medium outlet (13) in each compartment to adjust a separate temperature in each compartment, and
   - guiding a PCR liquid through the tube (6) and thereby through the separate temperatures in each compartment to prepare the DNA product based on the PCR liquid.
41. Method according to the preceding embodiment, wherein a first temperature in the first compartment (7) is in a range of about 85°C to about 105°C, preferably about 98°C.
42. Method according to one of the embodiments 40 to 41, wherein a second temperature in the second compartment (8) is in a range of about 45°C to about 72°C, preferably about 60°C to about 72°C.
43. Method according to one of the embodiments 40 to 42, further comprising providing a third compartment (15), wherein the tube (6) is wound at least from the second compartment (8) to the third compartment (15), and wherein a third temperature in the third compartment (15) is in a range of about 65°C to about 75°C, preferably about 72°C.
44. Method according to one of the embodiments 40 to 43, wherein the first temperature in the first compartment (7) is configured for a denaturation step of the Polymerase Chain Reaction, wherein the second temperature in the second compartment (8) is configured for an annealing step of the Polymerase Chain Reaction and optionally also for an elongation step of the Polymerase Chain Reaction.
45. Method according to embodiment 43, wherein the third temperature in the third compartment (15) is configured for an elongation step of the Polymerase Chain Reaction.
46. Use of a device according to one of the embodiments 1 to 26 for preparing a DNA product by means of Capillary Polymerase Chain Reaction.
47. Use of a manufacturing device (10) according to one of the embodiments 27 to 38 for a production of a pharmaceutical product.

A set of preferred embodiments of the present application relates to:
1. A device (1) for amplifying DNA by Polymerase Chain Reaction (PCR), comprising:
   - a tube (6) for guiding a PCR liquid,
   - a first compartment (7),
   - a second compartment (8), and
   - a third compartment (15),

   wherein the tube (6) is wound through the first compartment (7) and from the first compartment (7) to and through the second compartment (8), from the second department (8) to and through the third compartment (15), and from the third compartment (15) to the first compartment (7), wherein the tube (6) wound from compartment to compartment forms a helical stack of tube windings and each tube winding represents one PCR cycle, wherein the tube (6) comprises a PCR liquid inlet (20) and a DNA product outlet (25), and
   wherein the first compartment (7), the second compartment (8) and the third compartment (15) each comprises means to adjust the temperature in the compartment to prepare the DNA product based on the PCR liquid, wherein the first compartment is configured to provide a temperature for denaturation, the second compartment (8) is configured to provide a temperature for annealing and the third compartment (15) is configured to provide a temperature for elongation.
2. The device (1) for amplifying DNA according to embodiment 1, wherein the PCR liquid inlet (20) is connectable to a pump unit for pumping the PCR liquid through the tube (6).
3. The device (1) for amplifying DNA according to embodiment 1 or 2, wherein septum walls (14) are arranged between the compartments to insulate the compartments from each other, wherein the septum walls (14) comprise through holes for the tube (6) to extend from one compartment to the next.
4. The device (1) for amplifying DNA according to any one of embodiments 1 to 3, wherein each compartment comprises a heating unit (31) and optionally a cooling unit (32, 33) as the means to adjust the temperature in each compartment.
5. The device (1) for amplifying DNA according to any one of embodiments 1 to 3, wherein each compartment comprises a thermal medium inlet (12) and a thermal medium outlet (13) as the means to adjust the temperature in each compartment, namely to provide a separate thermal medium flow through the first compartment (7), the second compartment (8) and the third compartment (15) to adjust a separate temperature in the first compartment (7), the second compartment (8) and the third compartment (15).
6. The device (1) for amplifying DNA according to any one of the preceding embodiments, wherein the tube (6) extends within each of the compartments in a corrugated manner.
7. The device (1) for amplifying DNA according to any one of the preceding embodiments, wherein each compartment forms a basin to be filled with a thermal medium or a thermal solid.
8. The device (1) for amplifying DNA according to any one of the preceding embodiments, further comprising at least a stand (17) arranged in one of the compartments to provide a fixation point for the tube (6) and/or a deviation point for a corrugation of the tube (6).
9. The device (1) for amplifying DNA according to embodiment 8, wherein the stand (17) is exchangeable and/or repositionable in the compartment.
10. The device (1) for amplifying DNA according to any one of the preceding embodiments, wherein the tube (6) has a diameter in a range of about 0.5 mm to about 50 mm, preferably about 0.5 mm to about 1.5 mm, more preferably about 0.75 to about 1.25 mm.
11. The device (1) for amplifying DNA according to any one of the preceding embodiments, wherein the tube (6) has a length between a tube inlet and a tube outlet in a range of about 10 m to about 200 m, preferably about 50 m to about 100 m.
12. The device (1) for amplifying DNA according to any one of the preceding embodiments, wherein the tube (6) is dimensioned to contain PCR liquid in a range of about 10 mL to about 500 mL, preferably about 20 mL to about 100 mL.
13. The device (1) for amplifying DNA according to any one of the preceding embodiments, wherein the helical stack of tube windings comprises about 10 to about 50 windings, preferably about 15 to about 40.
14. A manufacturing device (10) comprising a device (1) for amplifying DNA according to any one of embodiments 1 to 13.
15. The manufacturing device (10) according to embodiment 14, further comprising a process chamber (2) and a technical chamber (3).
16. The manufacturing device (10) according to embodiment 15, further comprising a control unit configured to control a gas flow through the process chamber (2) as a gas shower.
17. The manufacturing device (10) according to embodiment 15 or 16, further comprising a separation element (4) separating the process chamber (2) from the technical chamber (3), wherein the separation element (4) comprises an appendix (16) with an opening towards the process chamber (2) and protruding in the direction of the technical chamber (3), and wherein the device (1) for amplifying DNA is insertable into the appendix (16).
18. The manufacturing device (10) according to embodiment 17, wherein a front face of the device (1) for preparing a DNA product ends essentially flush with the opening of the appendix (16) into the process chamber (2) when the device (1) for preparing a DNA product is inserted into the appendix (16).
19. A manufacturing module (100), comprising a manufacturing device (10) according to any one of embodiments 14 to 18 and at least one manufacturing device selected from the group consisting of a manufacturing device (10) comprising a process media supply unit and a mixing unit, a manufacturing device (10) comprising a purification unit, and a manufacturing device (10) comprising a filtration unit.
20. A method for amplifying DNA by means of Polymerase Chain Reaction (PCR), comprising:
   - providing a first compartment (7), a second compartment (8), a third compartment (15) and a tube (6), wherein the tube (6) is wound through the first compartment (7) and from the first compartment (7) to and through the second compartment (8) and from the second compartment (8) to and through the third compartment (15), and from the third compartment (15) to the first compartment (7), wherein the tube (6) wound from compartment to compartment forms a helical stack of tube windings,
   - providing means to adjust a separate temperature in each compartment, and
   - guiding a PCR liquid through the tube (6) and thereby through the separate temperatures in each compartment to prepare the DNA product based on the PCR liquid.
21. The method according to embodiment 20, wherein the temperature in the first compartment (7) is for denaturation, preferably in the range of about 85°C to about 105°C, preferably about 98°C.
22. The method according to embodiment 20 or 21, wherein the temperature in the second compartment (8) is for annealing, preferably in the range of about 45°C to about 72°C, preferably about 60°C to about 72°C.
23. The method according to any one of embodiments 20 to 22, wherein the temperature in the third compartment (15) is for elongation, preferably in the range of about 65°C to about 75°C, preferably about 72°C.
24. Use of a device (1) according to any one of embodiments 1 to 13 for preparing a DNA product by means of Polymerase Chain Reaction.
25. Use of a manufacturing device (10) according to any one of embodiments 14 to 18 or of a manufacturing module (100) according to embodiment 19 for the production of a pharmaceutical product.

A set of particularly preferred embodiments of the present application relates to:
1. A device (1) for preparing a DNA product by means of Capillary Polymerase Chain Reaction (PCR), comprising:
   - a tube (6) for guiding a PCR liquid,
   - a first compartment (7), and
   - at least a second compartment (8),

   wherein the tube (6) is wound at least from the first compartment (7) to the second compartment (8) and from the second compartment (8) to the first compartment (7), wherein the tube (6) wound from compartment to compartment forms a helical stack of tube windings, and
   wherein the first compartment (7) and the second compartment (8) each comprises means to adjust the temperature in the compartment to prepare the DNA product based on the PCR liquid, wherein the first compartment (7) is configured to provide a temperature for denaturation and the second compartment (8) is configured to provide a temperature for annealing and/or elongation.
2. The device (1) for preparing a DNA product according to embodiment 1, comprising
   - a first compartment (7),
   - a second compartment (8), and
   - a third compartment (15),

   wherein the tube (6) is wound from the first compartment (7) to the second compartment (8), from the second compartment (8) to the third compartment (15), and from the third compartment (15) to the first compartment (7),
   wherein the first compartment (7), the second compartment (8) and the third compartment (15) each comprises means to adjust the temperature in the compartment to prepare the DNA product based on the PCR liquid, wherein the first compartment (7) is configured to provide a temperature for denaturation, the second compartment (8) is configured to provide a temperature for annealing and the third compartment (15) is configured to provide a temperature for elongation.
3. The device (1) for preparing a DNA product according to embodiment 2, wherein the tube (6) is wound through the first compartment (7) and from the first compartment (7) to and through the second compartment (8), from the second compartment (8) to and through the third compartment (15), and from the third compartment (15) to the first compartment (7).
4. The device (1) for preparing a DNA product according to any one of embodiments 1 to 3, wherein each tube winding represents one PCR cycle.
5. The device (1) for preparing a DNA product according to any one of embodiments 1 to 4, wherein the tube (6) comprises a PCR liquid inlet (20) and a DNA product outlet (25).
6. The device (1) for preparing a DNA product according to embodiment 5, wherein the PCR liquid inlet (20) is connectable to a pump unit for pumping the PCR liquid through the tube (6).
7. The device (1) for preparing a DNA product according to embodiment 5, wherein the DNA product outlet (25) is for a continuous preparation of the DNA product or wherein the DNA product outlet (25) is a closable DNA product outlet (25) for a discontinuous preparation of the DNA product.
8. The device (1) for preparing a DNA product according to any one of the preceding embodiments, wherein septum walls (14) are arranged between the compartments to insulate the compartments from each other.
9. The device (1) for preparing a DNA product according to embodiment 8, wherein the septum walls (14) comprise through holes for the tube (6) to extend from one compartment to the next.
10. The device (1) for preparing a DNA product according to any one of the preceding embodiments, wherein each compartment comprises a heating unit (31) and optionally a cooling unit (32, 33) as the means to adjust the temperature in each compartment.
11. The device (1) for preparing a DNA product according to any one of embodiments 1 to 9, wherein each compartment comprises a thermal medium inlet (12) and a thermal medium outlet (13) as the means to adjust the temperature in each compartment, namely to provide a separate thermal medium flow through the first compartment (7), the second compartment (8) and the optional third compartment (15) to adjust a separate temperature in the first compartment (7), the second compartment (8) and the optional third compartment (15).
12. The device (1) for preparing a DNA product according to embodiment 11, wherein the thermal medium inlet (12) and the thermal medium outlet (13) are arranged within each compartment to provide the thermal medium flow through the compartment essentially in a counter direction to a guiding direction of the PCR liquid in the tube (6).
13. The device (1) for preparing a DNA product according to embodiment 11 or 12, wherein the thermal medium inlet (12) is arranged at a lower position in each of the compartments and the thermal medium outlet (13) is arranged at an upper position in each of the compartments.
14. The device (1) for preparing a DNA product according to any one of the preceding embodiments, wherein the tube (6) extends within at least one and preferably in each of the compartments in a corrugated manner.
15. The device (1) for preparing a DNA product according to any one of the preceding embodiments, wherein a stacking direction of the helical stack of tube windings is different and preferably essentially perpendicular to a corrugation direction of the corrugated tube (6).
16. The device (1) for preparing a DNA product according to any one of the preceding embodiments, wherein each compartment forms a basin to be filled with a thermal medium or a thermal solid.
17. The device (1) for preparing a DNA product according to any one of the preceding embodiments, further comprising at least a stand (17) arranged in one of the compartments to provide a fixation point for the tube (6) and/or a deviation point for a corrugation of the tube (6).
18. The device (1) for preparing a DNA product according to embodiment 17, wherein the tube (6) extends partially or fully or more than once around an outer dimension of the stand (17).
19. The device (1) for preparing a DNA product according to embodiment 17 or 18, wherein the stand (17) is exchangeable and/or repositionable in the compartment.
20. The device (1) for preparing a DNA product according to embodiment 6, wherein the pump unit is arranged outside the compartments and preferably outside a shell (26) surrounding the compartments.
21. The device (1) for preparing a DNA product according to embodiment 6 or 20, wherein the pump unit is configured to provide a flow rate of the PCR liquid in a range of about 0.1 to about 10 mL/min, preferably about 0.2 to about 3 mL/min.
22. The device (1) for preparing a DNA product according to any one of the preceding embodiments, further comprising at least a sensor (21) arranged in at least one of compartments, wherein the sensor (21) is configured to detect a temperature, a flow rate or a leakage of the compartment.
23. The device (1) for preparing a DNA product according to any one of the preceding embodiments, wherein the tube (6) has a diameter in a range of about 0.5 mm to about 50 mm, preferably 0.5 mm to about 1.5 mm, more preferably about 0.75 mm to about 1.25 mm.
24. The device (1) for preparing a DNA product according to any one of the preceding embodiments, wherein the tube (6) has a length between a tube inlet and a tube outlet in a range of about 10 m to about 200 m, preferably about 25 m to about 50 m.
25. The device (1) for preparing a DNA product according to any one of the preceding embodiments, wherein the tube (6) is dimensioned to contain PCR liquid in a range of about 10 mL to about 1 L, preferably about 20 mL to about 250 mL.
26. The device (1) for preparing a DNA product according to any one of the preceding embodiments, wherein the helical stack of tube windings comprises about 10 to about 50 windings, preferably about 15 to about 40.
27. The device (1) for preparing a DNA product according to any one of the preceding embodiments, wherein the device (1) for preparing a DNA product is configured to prepare about 1 mg and more of the DNA product per reaction, preferably in a range of about 1 to about 30 mg, more preferably in a range of about 15 to about 25 mg.
28. The device (1) for preparing a DNA product according to any one of the preceding embodiments, wherein the device (1) for preparing a DNA product is configured to prepare the DNA product in a range of about 50 µg to about 150 µg per mL PCR liquid, preferably about 75 µg to about 125 µg per mL PCR liquid.
29. A manufacturing device (10) for a pharmaceutical product comprising a device (1) for preparing a DNA product by means of Capillary Polymerase Chain Reaction according to any one of the preceding embodiments.
30. The manufacturing device (10) for a pharmaceutical product according to embodiment 29, further comprising a process chamber (2) and a technical chamber (3).
31. The manufacturing device (10) for a pharmaceutical product according to embodiment 30, further comprising a control unit configured to control a gas flow through the process chamber (2) as a gas shower.
32. The manufacturing device (10) for a pharmaceutical product according to embodiment 30 or 31, wherein at least the process chamber (2), preferably the manufacturing device (10), is operable according to the requirements of the EU guidelines for good manufacturing practice (GMP) for medicinal products, annex 1.
33. The manufacturing device (10) for a pharmaceutical product according to any one of embodiments 30 to 32, further comprising a separation element (4) separating the process chamber (2) from the technical chamber (3), wherein the separation element (4) comprises an appendix (16) with an opening towards the process chamber (2) and protruding in the direction of the technical chamber (3), and wherein the device (1) for preparing a DNA product is insertable into the appendix (16).
34. The manufacturing device (10) for a pharmaceutical product according to embodiment 33, wherein a front face of the device (1) for preparing a DNA product ends essentially flush with the opening of the appendix (16) into the process chamber (2) when the device (1) for preparing a DNA product is inserted into the appendix (16).
35. The manufacturing device (10) for a pharmaceutical product according to any one of embodiments 29 to 34, wherein at least a handle (22) for handling the device (1) for preparing a DNA product in and/or out of the appendix (16) is positioned at the front face of the device (1) for preparing a DNA product.
36. The manufacturing device (10) for a pharmaceutical product according to any one of embodiments 29 to 35, wherein an intake for a PCR liquid (23) and/or an outfall for a DNA product (24) is/are positioned at the front face of the device (1) for preparing a DNA product.
37. The manufacturing device (10) for a pharmaceutical product according to any one of embodiments 29 to 36, wherein an intake for a thermal medium and/or an outfall for the thermal medium is/are positioned at a back plate of the device (1) for preparing a DNA product opposite to the front face of the device (1) for preparing a DNA product.
38. The manufacturing device (10) for a pharmaceutical product according to embodiment 37, further comprising a leakage sensor (21) arranged in the appendix (16) outside the device (1) for preparing a DNA product.
39. The manufacturing device (10) for a pharmaceutical product according to any one of embodiments 29 to 36, wherein a ventilator (34) and/or an air inlet (35) is/are positioned at a back plate of the device (1) for preparing a DNA product opposite to the front face of the device (1) for preparing a DNA product.
40. The manufacturing device (10) for a pharmaceutical product according to any one of embodiments 29 to 39, wherein the appendix (16) comprises exchange rails (27) to guide an exchange of the device (1) for preparing a DNA product with another device (1) for preparing a DNA product.
41. The manufacturing device (10) for a pharmaceutical product according to any one of embodiments 29 to 40, wherein a pump unit for pumping the PCR liquid through a tube (6) of the device (1) for preparing a DNA product is arranged outside the device (1) for preparing a DNA product, preferably wherein a pump unit for pumping the PCR liquid through a tube (6) of the device (1) for preparing a DNA product is arranged within the process chamber of the manufacturing device (10).
42. The manufacturing device (10) for a pharmaceutical product according to any one of embodiments 29 to 41, further comprising a coupling unit (18) arranged at the casing of the manufacturing device (10) for a pharmaceutical product and configured to couple the manufacturing device (10) for a pharmaceutical product to another manufacturing device (10).
43. A manufacturing module (100) for a pharmaceutical product, comprising a manufacturing device (10) for a pharmaceutical product according to any one of embodiments 29 to 42 and at least one manufacturing device selected from the group consisting of a manufacturing device (10) comprising a process media supply unit and a mixing unit, a manufacturing device (10) comprising a purification unit, and a manufacturing device (10) comprising a filtration unit.
44. A method for preparing a DNA product by means of Capillary Polymerase Chain Reaction (PCR), comprising:
   - providing a first compartment (7), a second compartment (8), and a tube (6), wherein the tube (6) is wound at least from the first compartment (7) to the second compartment (8) and from the second compartment (8) to the first compartment (7), wherein the tube (6) wound from compartment to compartment forms a helical stack of tube windings,
   - providing means to adjust a separate temperature in each compartment, and
   - guiding a PCR liquid through the tube (6) and thereby through the separate temperatures in each compartment to prepare the DNA product based on the PCR liquid.
45. The method according to embodiment 44, wherein the temperature in the first compartment (7) is for denaturation, preferably in the range of about 85°C to about 105°C, preferably about 98°C.
46. The method according to embodiment 44 or 45, wherein the temperature in the second compartment (8) is for annealing and/or elongation, preferably in the range of about 45°C to about 72°C, preferably about 60°C to about 72°C.
47. The method according to any one of embodiments 44 to 46, further comprising providing a third compartment (15), wherein the tube (6) is wound from the second compartment (8) to the third compartment (15) and from the third compartment (15) to the first compartment (7), and wherein the temperature in the third compartment (15) is for elongation, preferably in the range of about 65°C to about 75°C, preferably about 72°C.
48. The method according to any one of embodiments 44 to 47, wherein each compartment comprises a heating unit (31) and optionally a cooling unit (32, 33) as the means to adjust a separate temperature in each compartment.
49. The method according to any one of embodiments 44 to 47, wherein each compartment comprises a thermal medium inlet (12) and a thermal medium outlet (13) as the means to adjust a separate temperature in each compartment, namely to provide a separate thermal medium flow through the first compartment (7), the second compartment (8) and the optional third compartment (15) to adjust a separate temperature in the first compartment (7), the second compartment (8) and the optional third compartment (15).
50. Use of a device (1) according to any one of embodiments 1 to 28 for preparing a DNA product by means of Capillary Polymerase Chain Reaction.
51. Use of a manufacturing device (10) according to any one of embodiments 29 to 42 or of a manufacturing module (100) according to embodiment 43 for the production of a pharmaceutical product.

## Claims

1. A device (1) for preparing a DNA product by means of Capillary Polymerase Chain Reaction (PCR), comprising:
- a tube (6) for guiding a PCR liquid,
- a first compartment (7), and
- at least a second compartment (8),
wherein the tube (6) is wound at least from the first compartment (7) to the second compartment (8) and from the second compartment (8) to the first compartment (7), wherein the tube (6) wound from compartment to compartment forms a helical stack of tube windings, and
wherein the first compartment (7) and the second compartment (8) each comprises means to adjust the temperature in the compartment to prepare the DNA product based on the PCR liquid, wherein the first compartment (7) is configured to provide a temperature for denaturation and the second compartment (8) is configured to provide a temperature for annealing and/or elongation.

2. The device (1) for preparing a DNA product according to claim 1, wherein each tube winding represents one PCR cycle.

3. The device (1) for preparing a DNA product according to claim 1 or 2, wherein the tube (6) comprises a PCR liquid inlet (20) and a DNA product outlet (25).

4. The device (1) for preparing a DNA product according to claim 3, wherein the PCR liquid inlet (20) is connectable to a pump unit for pumping the PCR liquid through the tube (6).

5. The device (1) for preparing a DNA product according to any one of the preceding claims, wherein septum walls (14) are arranged between the compartments to insulate the compartments from each other, and optionally the septum walls (14) comprise through holes for the tube (6) to extend from one compartment to the next.

6. The device (1) for preparing a DNA product according to any one of the preceding claims, wherein each compartment comprises a heating unit (31) and optionally a cooling unit (32, 33) as the means to adjust the temperature in each compartment.

7. The device (1) for preparing a DNA product according to any one of the preceding claims, wherein each compartment forms a basin to be filled with a thermal medium or a thermal solid.

8. The device (1) for preparing a DNA product according to claim 4, wherein the pump unit is configured to provide a flow rate of the PCR liquid in a range of about 0.1 to about 10 mL/min, preferably about 0.2 to about 3 mL/min.

9. The device (1) for preparing a DNA product according to any one of the preceding claims, wherein the tube (6) has a diameter in a range of about 0.5 mm to about 50 mm, preferably 0.5 mm to about 1.5 mm, more preferably about 0.75 mm to about 1.25 mm, and/or wherein the tube (6) has a length between a tube inlet and a tube outlet in a range of about 10 m to about 200 m, preferably about 25 m to about 50 m.

10. The device (1) for preparing a DNA product according to any one of the preceding claims, wherein the tube (6) is dimensioned to contain PCR liquid in a range of about 10 mL to about 1 L, preferably about 20 mL to about 250 mL.

11. The device (1) for preparing a DNA product according to any one of the preceding claims, wherein the helical stack of tube windings comprises about 10 to about 50 windings, preferably about 15 to about 40.

12. The device (1) for preparing a DNA product according to any one of the preceding claims, wherein the device (1) for preparing a DNA product is configured to prepare about 1 mg and more of the DNA product per reaction, preferably in a range of about 1 to about 30 mg, more preferably in a range of about 15 to about 25 mg, and/or wherein the device (1) for preparing a DNA product is configured to prepare the DNA product in a range of about 50 µg to about 150 µg per mL PCR liquid, preferably about 75 µg to about 125 µg per mL PCR liquid.

13. A manufacturing device (10) for a pharmaceutical product comprising a device (1) for preparing a DNA product by means of Capillary Polymerase Chain Reaction according to any one of the preceding claims, and optionally the manufacturing device (10) further comprising a process chamber (2) and a technical chamber (3).

14. A manufacturing module (100) for a pharmaceutical product, comprising a manufacturing device (10) for a pharmaceutical product according to claim 13 and at least one manufacturing device selected from the group consisting of a manufacturing device (10) comprising a process media supply unit and a mixing unit, a manufacturing device (10) comprising a purification unit, and a manufacturing device (10) comprising a filtration unit.

15. A method for preparing a DNA product by means of Capillary Polymerase Chain Reaction (PCR), comprising:
- providing a first compartment (7), a second compartment (8), and a tube (6), wherein the tube (6) is wound at least from the first compartment (7) to the second compartment (8) and from the second compartment (8) to the first compartment (7), wherein the tube (6) wound from compartment to compartment forms a helical stack of tube windings,
- providing means to adjust a separate temperature in each compartment, and
- guiding a PCR liquid through the tube (6) and thereby through the separate temperatures in each compartment to prepare the DNA product based on the PCR liquid.

16. Use of a device (1) according to any one of claims 1 to 12 for preparing a DNA product by means of Capillary Polymerase Chain Reaction.

17. Use of a manufacturing device (10) according to claim 13 or of a manufacturing module (100) according to claim 14 for the production of a pharmaceutical product.
